# EUROPEAN PATENT APPLICATION

(11) **EP 4 570 188 A1**
(43) Date of publication of application: **18.06.2025**
(21) Application number: 24219658.2
(22) Date of filing: 13.12.2024
(51) Int. Cl.: A61B 8/12, A61B 8/00

(54) **OVER-THE-WIRE MEDICAL TOOL POSITIONING DEVICES, SYSTEMS, AND METHODS OF USE AND MANUFACTURE**

(30) Priority: 15.12.2023 US 202318541745
(71) Applicant: NuVera Medical, Inc., Los Gatos CA 95032 (US)
(72) Inventor: SCHAER, Alan, Los Gatos, 95032 (US)
(74) Representative: Carpmaels & Ransford LLP

(57) **Abstract**

A medical device may comprise an ultrasound transducer in a distal region of the medical device, the ultrasound transducer secured to an outer shaft, the ultrasound transducer at least partially enclosed by a material to define a distal tip of the medical device. The medical device may comprise a tip extension disposed adjacent the distal tip and extended longitudinally from the distal tip, the tip extension comprising a first end adjacent the distal tip and a second end opposite the first end. The tip extension may comprise a tapered tip at or adjacent the second end. The tip extension may comprise a guidewire lumen formed therein and configured to receive a guidewire therethrough.

## Description

### INCORPORATION BY REFERENCE

This application incorporates by reference herein the entire disclosures of the following applications, which are incorporated by reference herein for all purposes: PCT/US2019/061228 filed November 13, 2019; U.S. Prov. App. No. 62/760,784 filed November 13, 2018; WO2018/017717, published 1/25/2018; US20220401070A1; and WO 2018/182836, published 10/4/2018.

All publications and patent applications mentioned in this specification are herein incorporated by reference to the same extent as if each individual publication or patent application was specifically and individually indicated to be incorporated by reference.

### BACKGROUND

A wide variety of intravascular medical devices are known. Improved systems, devices, and methods that facilitate better control, positioning, and usability of medical devices are needed.

### SUMMARY OF THE DISCLOSURE

The disclosure herein is related to medical devices and uses thereof. The disclosure is generally related to a medical device configured and sized to be positioned within a subject. The medical device may comprise an ultrasound transducer in a distal region of the medical device, the ultrasound transducer secured to an outer shaft, the ultrasound transducer at least partially enclosed by a material to define a distal tip of the medical device. The medical device may comprise a tip extension disposed adjacent the distal tip and extended longitudinally from the distal tip, the tip extension comprising a first end adjacent the distal tip and a second end opposite the first end. The tip extension may comprise a tapered tip at or adjacent the second end. The tip extension may comprise a guidewire lumen formed therein and configured to receive a guidewire therethrough. The tip extension may exhibit a stiffness transition. The tip extension may exhibit a first stiffness at the first end and a second stiffness adjacent the second end, wherein the first stiffness is greater than the second stiffness.

One aspect of the disclosure is a medical device that is sized and configured to be positioned within a subject, such as within a blood vessel, chamber of the heart, or other bodily lumen or space.

The medical devices herein may include a medical tool such as an ultrasound transducer in a distal region of the medical device. The medical tool may be secured (directly or indirectly) to a flexible member(s), such as flexible electronics (e.g., a flexible conductor bundle) at a first location. The flexible member may extend proximally towards (and optionally into) a handle assembly. In some examples, the disclosure is related to trying to prevent the flexible electronics from deforming to an undesirable extent and/or trying to maintain a configuration of the flexible electronics (even if there some minor degree of deformation).

The medical device may include one or more elongate shafts through which the flexible member extends. The medical device may include more than one elongate shaft through which the flexible member extends, such as an outer shaft and an inner shaft, the inner shaft extending through at least a portion of the outer shaft. An inner shaft may be movable relative to the outer shaft. An inner shaft may be independently deflectable relative to the outer shaft.

The flexible member (e.g., flexible conductor bundle) may be axially movable within the one or more elongate shafts, and may be fixed to a shaft, such as an outer shaft.

### BRIEF DESCRIPTION OF THE DRAWINGS

Figure 1A illustrates an example of a system that includes steering and a medical device.
Figure 1B illustrates a cross section A-A of the steering and device portion of the medical device of Figure 1A.
Figure 2 illustrates an example system that includes a handle assembly with a plurality of actuators, a steerable sheath and medical tool.
Figures 3Ai, 3Aii and 3Aiii illustrate an example steerable shaft with pull wires.
Figures 3Bi and 3Bii illustrate an example steerable shaft with pull wires.
Figures 3Ci and 3Cii illustrate an example steerable shaft with pull wires.
Figures 3Di - 3Div illustrate an example steerable shaft with pull wires.
Figure 3E illustrates an example steerable shaft with one or more pull wires circumferentially interwoven into braid wires of the shaft.
Figure 4 illustrates an example portion of an example system that includes a bundle.
Figure 5 illustrates an example proximal end of a medical tool, the tool including a conductor bundle that extends into a proximal connector within which is housed a printed circuit board (PCB).
Figure 6A illustrates a portion of an example medical tool that includes a flexible circuit strip.
Figure 6B illustrates an example proximal portion of a strip.
Figure 6C illustrates a detailed view of an example proximal portion of a strip.
Figure 6D illustrates an end view of an example flex strip.
Figure 6E illustrates an example stack of flex strips.
Figure 6F illustrates an example stack of flex strips and ground and shield strips.
Figure 6G illustrates an example bundle including a tubing material around a stack of strips and shield and ground strips.
Figure 7 illustrates an integrated system of the steerable sheath and medical tool wherein the system is connected to a console via a connector cable.
Figures 8A and 8B illustrate an example handle assembly that can be used with any of the inner and outer elongate bodies or shafts herein.
Figure 9A illustrates a portion of an example inner elongate body, or inner shaft.
Figure 9B illustrates a portion of an example outer elongate body, or outer shaft.
Figure 9C illustrates a portion of an example medical device including the elongate bodies (or shafts) from figures 9A and 9B.
Figure 9D illustrates a section of the device in the deflectable portion from figure 9C.
Figure 10A illustrates a portion of an example handle assembly.
Figure 10B is an exploded view illustrating an example outer elongate body (or outer shaft) movement subassembly.
Figure 10C illustrates a side sectional view of the handle assembly from figure 57A.
Figure 11 illustrates an example handle assembly that includes rotation indicators for first and second actuators.
Figure 12A illustrates internal components of an example handle assembly that includes a tensioning member, or flattening member.
Figure 12B illustrates (in a side view) internal components of an example handle assembly that includes a tensioning member, or flattening member.
Figure 12C illustrates (in a side view that is the other side relative to figure 12B) internal components of an example handle assembly that includes a tensioning member, or flattening member.
Figure 12D illustrates internal components of an example handle shell that include one or more guiding features positioned to help stabilize a tensioning member in the handle assembly.
Figure 12E illustrates a side view of an example handle shell.
Figure 12F illustrates a side view of an example handle shell.
Figure 13A illustrates an example flexible cable bundle with at least a portion that is twisted relative to a long axis.
Figure 13B illustrates an example flexible cable bundle with at least a portion that is twisted relative to a long axis.
Figure 13C illustrates an example flexible cable bundle with at least a portion that is twisted relative to a long axis.
Figure 13D illustrates an example flexible cable bundle with at least a portion that is twisted relative to a long axis.
Figure 13E illustrates a detail from figure 13D.
Figures 14-18 illustrate example medical devices with navigation interface.
Figures 19-24 illustrate example transducer tips for medical devices.
Figure 25 illustrates an example assembly for a medical device.
Figure 26 illustrates an arrangement of navigation sensors for a medical device.
Figures 27-29 illustrate various configurations of pull wires used to deflect a portion of a shaft or sheath of a medical device.
Figures 30-31 illustrate various arrangements of components in a handle assembly of a medical device.
Figures 32 and 33A-33B illustrate example medical devices with tip extensions.

### DETAILED DESCRIPTION

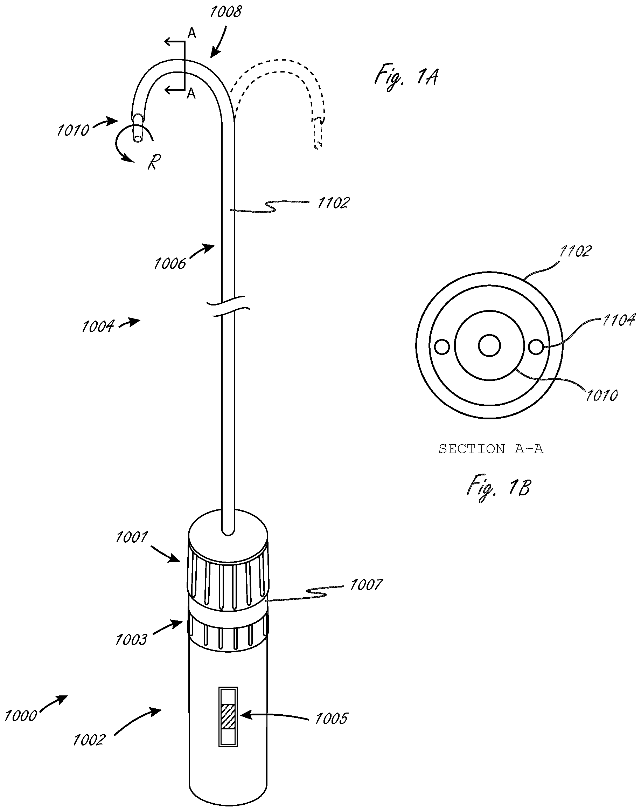

Figure 1A illustrates an example of a system that integrates steering and a medical device. System 1000 includes handle assembly 1002 and steering and medical device portion 1004. Steering and medical device portion 1004 includes a proximal portion 1006 and steerable portion 1008. The system is adapted so that handle assembly 1002 can be actuated to cause steering of the steerable portion 1008, and optionally can be further actuated to cause movement of medical device 1010 relative to steering and medical device portion 1004. In this example, handle assembly 1002 includes first actuator 1001, second actuator 1003, and third actuator 1005. First actuator 1001 is adapted to be actuated (in this example rotated) relative to handle body 1007 to cause the steering of steerable portion 1008, and specifically steering outer sheath 1102. Steerable portion 1008 may be steered, or bent, into the configuration shown in Figure 1A in solid lines, and can also be steered into the configuration shown in dashed lines, or anywhere in between, and in some examples the opposite steering function is limited to simply straightening the shaft from an initial bent configuration, such as the solid line bent configuration in Figure 1A. The term "steer" in this disclosure means to deflect or bend, optionally via actuation of at least one pull wire, but in some instances the term can include shaft rotation (torqueing) and axial movement. The term "pull wire" herein refers to any element that may transmit a tensile force from the proximal end of the device to the distal end region. Pull wires may be comprised of metal wire such as stainless steel or nickel titanium, either solid or stranded/braided, or it may be comprised of a polymer such as aramid fiber (Kevlar^{®}), polyethylene, ptfe, eptfe, etc., preferably stranded/braided, but also in monofilament form. In an example, the pull wire is constructed from an aramid fiber bundle having four 50 denier multifilament (approximately 25 filaments) threads braided together at a high picks per inch. The wire cross-sectional diameter is typically in the .005"-.012" range, more preferably .008"-.010", although braided or stranded wire may flatten or ovalize in the device lumen. Certain examples are believed to provide optimized strength and wear resistance for the size necessary to keep the shaft diameters to a minimum. Optional second actuator 1003 is adapted to be actuated relative to handle body 1007 (in this example rotated) to cause rotation of medical tool 1010 relative to shaft 1102 (labeled as rotation movement "R"), and optional actuator 1005 is adapted to be actuated relative to handle body 1007 (in this example axially) to cause axial (distal-proximal) movement of medical device 1010 relative the outer sheath 1102. Proximal portion 1006 is not configured to bend significantly when steerable portion 1008 is steered (bent/deflected), although the proximal portion may flex and bend to conform to the anatomy within which it is used. In many aspects, this is accomplished by constructing the steerable portion 1008 from a softer or less rigid material and/or composite construction than the proximal portion 1006.

The example shown in Figure 1A is an example of an apparatus that includes an integrated handle assembly that is in operable communication with both a steerable outer shaft and an inner medical tool. The handle assembly is integrated in that it is assembled and constructed to be in operable communication with the outer shaft and the inner medical tool prior to packaging and use. "Integrated" as that term is used in the context of an integrated handle assembly refers to a handle assembly in which at least one part of the handle assembly has to be broken or taken apart before the medical tool can be removed from within the outer shaft.

Figure 1B illustrates an example cross section A-A (shown in Fig. 1A) of the steering and device portion 1004, and specifically in the steerable portion 1008. In this example medical device 1010 is sized and configured to be disposed within a steerable sheath. The steerable sheath includes an outer shaft 1102 and a set of pull wires 1104, which are axially fixed in a distal region of steerable portion 1008.

The medical tool in Figures 1A and 1B can be, for example, any medical tool herein, such as an ultrasound tool. When "ultrasound probe" is used herein, it generally refers to an elongate tool that includes at least one ultrasound transducer and one or more conductive elements that electrically connect the at least one ultrasound transducer to a proximal region of the elongate tool. A proximal region of the ultrasound probe includes, or is modified to include, at least one proximal contact, which is in electrical communication with the at least one ultrasound transducer, and which can be put into electrical communication with, optionally via attachment to, an electrical contact on another device, cable, or connector.

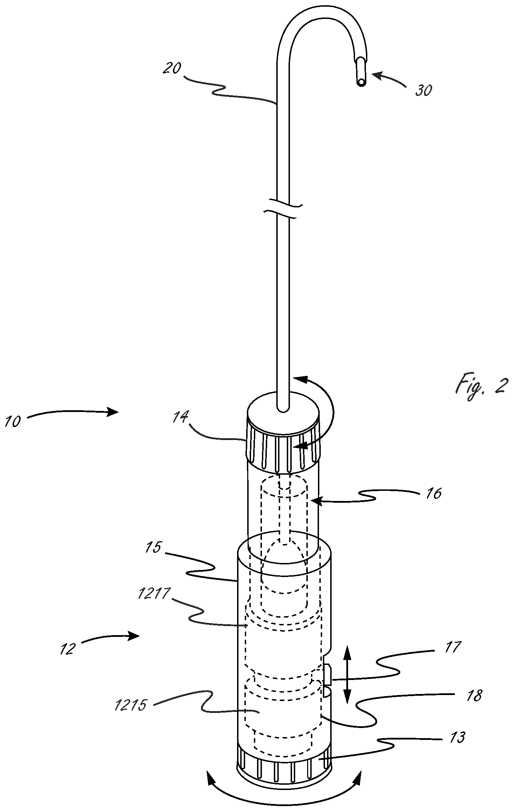

Figure 2 illustrates an example system 10 that is adapted to function similarly to the system in figures 1A and 1B, and also illustrates example internal components of handle assembly 12 (internal components shown as dashed lines). Handle assembly 12 is integrated and in operable communication with outer steerable shaft 20 and medical tool 30. Handle assembly 12 includes actuator 14 that is adapted to, when actuated relative to handle body 15, cause steering of steerable shaft 20. Actuator 14 is in operable communication with steerable shaft 20 via steering control 16 disposed in handle assembly 12. Medical tool 30 includes a proximal portion 18 disposed within and incorporated into handle assembly 12. Actuator 13 is in operable communication with medical tool 30, and actuation of actuator 13 (in this example rotation) relative to handle body 15, causes rotation of medical tool 30 relative to outer shaft 20 via rotation control 1215. Optional third actuator 17 is also in operable communication with medical tool 30, and is adapted to be actuated, in this example, axially (relative to handle body 15), to cause axial movement of medical tool 30 relative to outer steerable shaft 20 via axial control 1217.

The medical tool in Figure 2 can be, for example, any medical tool herein, such as an ultrasound tool.

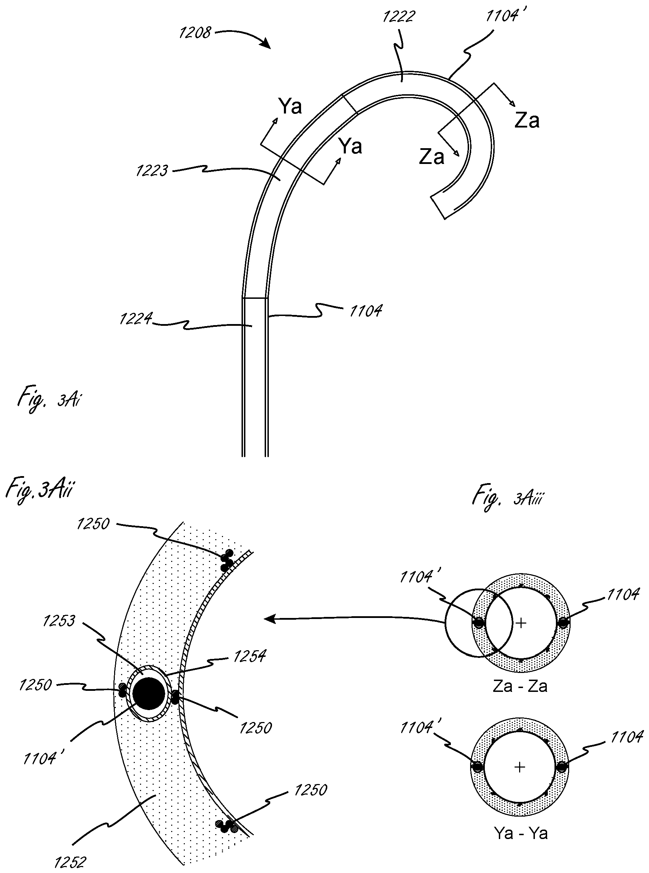

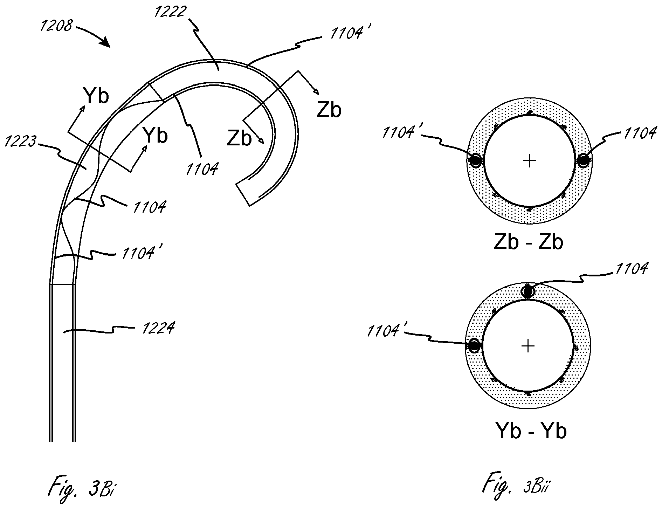

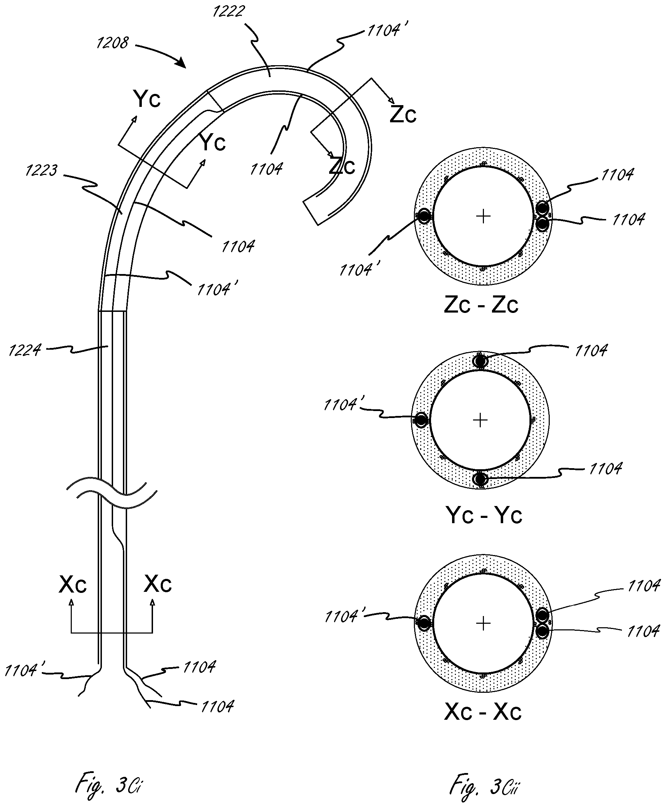

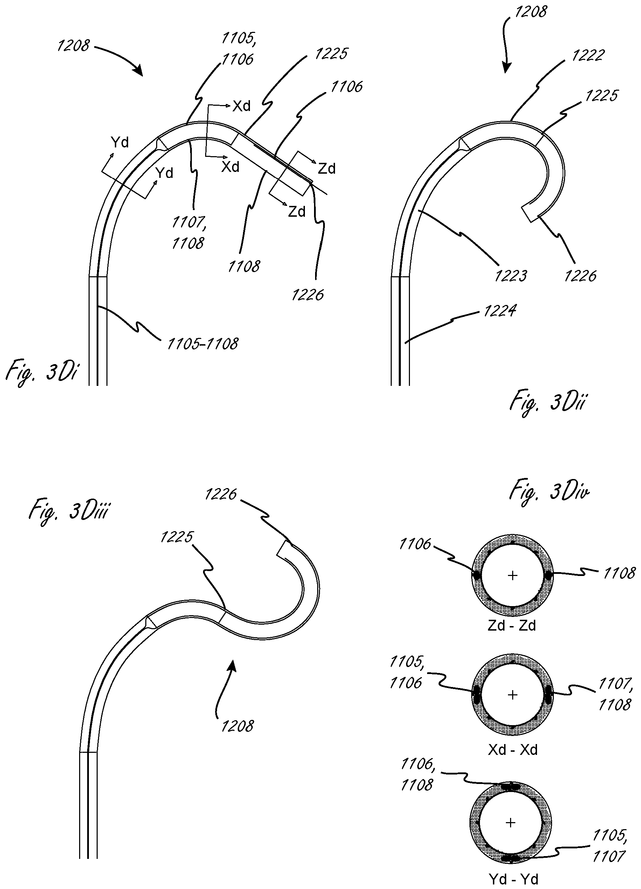

Figures 3A-3E represent example examples of a distal region of the sheath portion 1208 of steerable sheath 1202 in system 1200. For simplicity, the illustrated cross-sections show only the outer sheath 1208 and not the inner tool 1212. The outer sheath 1208 preferably has a composite construction to improve torque transmission applied to the outside of the shaft from the proximal end, or to resist torque forces applied to it from within the shaft, such as from tool 1212. As illustrated in Figure 3Ai-iii, in order to form the composite, multiple braid elements 1250, preferably formed from metal wire (round, pairs of round, or ribbon shaped) and/or multiple fibers (e.g., aramid or nylon), may be braided directly over a thin wall (e.g., .0010" ± .0005") lubricious liner tube 1251, such as a PTFE or FEP material. A thermoplastic polymer 1252 (such as Pebax in a range of durometers from 25D-72D, or nylon, or other common catheter materials) may be laminated with heat using heat shrink tubing (such as FEP) to reflow the polymer over the braid elements 1250 and liner tube 1251 to form a uniform member. The thermoplastic polymer 1252 may also have radiopaque compounds that include materials such as bismuth, barium sulfate, or tungsten in order that the tip of the sheath be visible to the user under fluoroscopy. In the example of Figure 3Ai-iii, the pull wire 1104 is preferably parallel to the central access in the steerable (deflectable) portion 1222 of the sheath and also preferably provided in a lumen 1253 created within the wall of the steerable sheath 1208. This lumen may be created during the thermoplastic polymer tubing extrusion process or during a shaft heat lamination fusing process with the aid of a removable mandrel. The pull wire lumen 1253 may further be created by incorporating a pull wire tube 1254, preferably temporarily supported by a removable mandrel, within the wall. The removable mandrel may also be placed alongside the pull line 1104 or 1104' during the fusing process, resulting in a somewhat ovalized lumen 1253 within which a fiber pull wire may be allowed to flatten into, allowing space for free movement of the pull wire. The tube 1254 may include PTFE, FEP, polyimide, or another material which maintains its wall integrity during a heat lamination process up to approximately 500 °F. The tube is preferably surrounded and supported by the thermoplastic polymer 1252 which is preferably heat laminated against the tube. In another example, the pull wire lumen, preferably comprising the pull wire tube, is incorporated within the weave of the braid elements 1250. For example, braid elements 1250 running in one direction would pass under the pull wire lumen, while those running in the opposite direction would pass over the pull wire lumen. The braid reinforcement provides a more dimensionally stable lumen during catheter manipulations and also helps assure the straightness of the lumen as needed. Proximal to the steerable portion, the pull wire may continue proximally parallel to the central axis on the same side of the outer sheath 1208, such as is illustrated in Figure 3Ai-iii. In this example and others that follow, an additional pull wire 1104' within an additional pull wire lumen routed within the wall of sheath 1208, up through the steerable portion 1222, may be required to straighten the steerable portion of the device. This straightening pull wire 1104' is preferably routed within steerable portion 1222 on the side opposite from the pull wire(s) 1104 used for steering (deflection) in the steerable portion 1222. In another example, not shown, two lumens and two straightening pull wires 1104' could be used, essentially mirroring the paired 1104 pull wire configuration. These straightening wires could also be constructed to allow deflection in the opposite direction by tensioning a greater distance (beyond just straightening) within the handle.

During use, a portion 1223 of the distal catheter just proximal to the steerable (deflectable) portion 1222 may be forced to conform to a curve based on the constraints of the anatomy in which it is used. For a specific example where the device is advanced into the heart chambers from a groin access, the portion 1223 forced into a curve is expected to range from 5 to 25 cm in length. During rotation of the sheath shaft 1208 from the proximal end, torque is transmitted through this distal curved region 1223 to the catheter tip. A non-uniform cross section and/or tension of the device in this region 1223 may induce a tendency for the shaft to build up and suddenly release torque, causing a "whip" or sudden jerk in rotation as it is torqued. To minimize the potential for whip, it is optional to distribute the pull wire tension and construction material around the surface of the curved region 1223. In one example, such as is illustrated in Figure 3Bi-iii, the pull wire 1104 may spiral around the central axis of the sheath in at least the curved region 1223 proximal to portion 1222. The pull wire of this example may make a full circumferential wrap over approximately 10 cm of length, with this value ranging 5-15 cm. The spiral may only need to be present in the curved region 1223, continuing straight proximally thereafter through proximal portion 1224 (similar to 1006), which may minimize the friction in the pull wire lumen and the associated pull wire force required to steer (deflect) the steerable portion 1222. The spiral may also make a minimum of one turn before continuing straight, or spiral the full length of the shaft. In another example to minimize whip, it may only be necessary to distribute the pull wire tension to opposite sides of the shaft. As illustrated in Figure 3Ci-ii, deflection of the steerable section 1222 is accomplished with two parallel pull wires 1104 positioned adjacent one another on the same side of the sheath 1208. In the curved region 1223 and proximal portion 1224 (similar to 1006) proximal to the steerable section 1222, the pull wires are routed to opposite sides of the shaft, each 90° from the position in the steerable section 1222, to distribute the tension more evenly. While it is preferable to actuate the two parallel pull wires at the same time with equal force with the handle actuator, in other examples, a differential in force could be applied to steer the tip to one side or the other of the plane formed when the two are actuated with equal force. In other examples, any plurality of pull wires could be routed in the same configuration as illustrated in Figures 3B or Figure 3C, with the multiple proximal pull wires distributed uniformly around the shaft circumference. Also, as illustrated in Figure 3Ci-ii, the pull wires 1104 may be routed proximally along the opposite sides of the shaft for most of the shaft proximal portion 1124 length, but preferably brought back together adjacent one another near the proximal end portion of the shaft to allow the wires to exit the same side of the proximal shaft together to facilitate them being secured together to a handle component for simultaneous actuation tension.

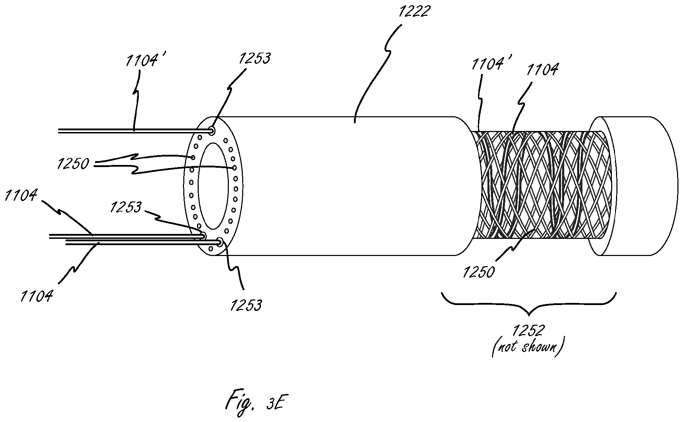

Figures 3Di-iv illustrate another example of the distal region of catheter with construction similar to that previously described, but instead configured to provide a distal steerable portion 1222 which can be deflected into two different directions. As illustrated, a two pairs of pull wires 1105/1107 and 1106/1108 are along the proximal shaft region 1224 and curved region 1223. This is similar to Figure 3Ai-iii, except that the wires are paired on each side of the shaft. The routing could also be spiraled as in Figure 3Bi-ii, or other configurations discussed. Within distal steerable portion 1222, the wires are routed 90° from the proximal portions, although other angles are contemplated. At a junction 1225 within 1222 one or more of the pull wires (e.g., 1105 and 1107) may be terminated and anchored to the shaft, with the remaining pull wires (e.g., 1106 and 1108) continuing to a more distal tip location 1226 where they are anchored. This configuration allows independent actuation of pull wires terminated at 1225 and 1226 such that different shapes may be created during actuation. Figure 3Dii shows both lines 1107 and 1108 tensioned to create a variable curve in the same direction. Figure 3Diii shows lines 1107 and 1106 tensioned to create an "S" curve. Other configurations are also possible.

The pull wires (such as 1104 and 1104') must be terminated at their distal end in a manner that reliably affixes them to the wall of the distal steerable shaft portion 1222, such that they do not break or pull free under repeated applications of tension. In an example, shown in Figure 3E, the pull wires 1104 and 1104', upon exiting the distal pull wire lumen 1253, are circumferentially interwoven into the braid wires 1250 of the distal shaft 1222 (shown without the thermoplastic polymer 1252). One or more of the pull wires 1104 or 1104' may also be additionally or instead wrapped and/or tied around the outside of the braid wires 1250 for additional securing. The braid wires 1250 may be then trimmed distal to the securing point, with the interwoven and/or wrapped pull wires preventing the braid wires from expanding and/or unraveling. Additional adhesives such as UV cured or cyanoacrylates may also be used to secure the pull wires to the braid wires. The weave and/or wrap of the pull wires and braid wires is then laminated with a thermoplastic polymer which melts within the space around the wires and cools to secure them in place. The thermoplastic polymer may also have radiopaque compounds that include materials such as bismuth, barium sulfate, or tungsten in order that the tip of the sheath be visible to the user under fluoroscopy.

In additional examples, the tool 1212 may also or alternatively be constructed with one or more pull wires to deflect the tip in a manner similar to any of the previous examples described for the outer sheath 1208. In addition to routing the pull wires within the wall of the tubular member of the tool 1212, the pull wires could be routed next to the conductors inside the lumen of the tubular element 1212. Actuation of the pull wires could be from an actuator located in the proximal handle 1206. The distal shaft of tool 1212 may also be formed into a particular shape (e.g., an arc) such that it bends into the shape as it exits the tip of the steerable portion 1222 of outer sheath 1208. The stiffness of the distal shaft of tool 1212 is such that it does not substantially deform outer sheath 1208 while inside, but upon exiting is allowed to bend. The shape may be set by any one or combination of the following means: heat setting the polymeric material, using a moveable or fixed shaped stylet within the inner lumen of shaft 1212 or within a lumen within the wall of shaft 1212. Such a stylet could be round, oval, or rectangular in cross section, and be formed of stainless steel, nitinol, or a rigid polymer such as PEEK, Vestamid, or similar. The outer steerable sheath could alternatively be made to bend with a similar method as above, with or without additional pull wire deflection, and with or without additional shape or deflection of the distal portion of tool shaft 1212.

One aspect of the disclosure includes methods of disassociating at least a portion of the system from other components, optionally as part of a reposing process. In some examples the medical tool includes one or more electrical contacts that are coupled to other electrical contacts, which are in electrical communication with an energy console, and examples of consoles are known in the ultrasound art.

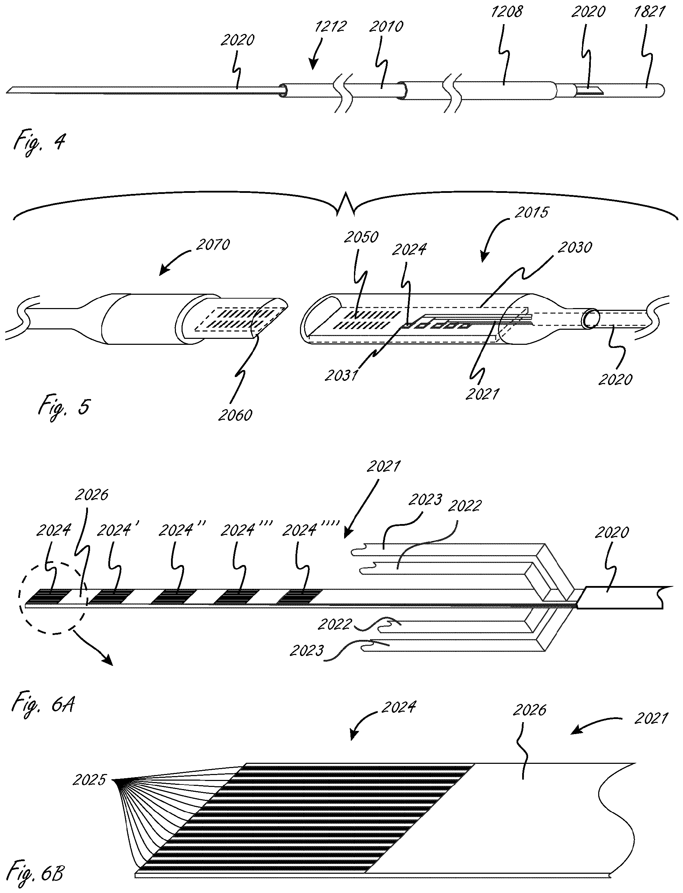

Figure 4 illustrates merely a portion of an example medical tool, such as an ultrasound probe, that can be electrically coupled directly or indirectly to an energy console, such as an ultrasound console.

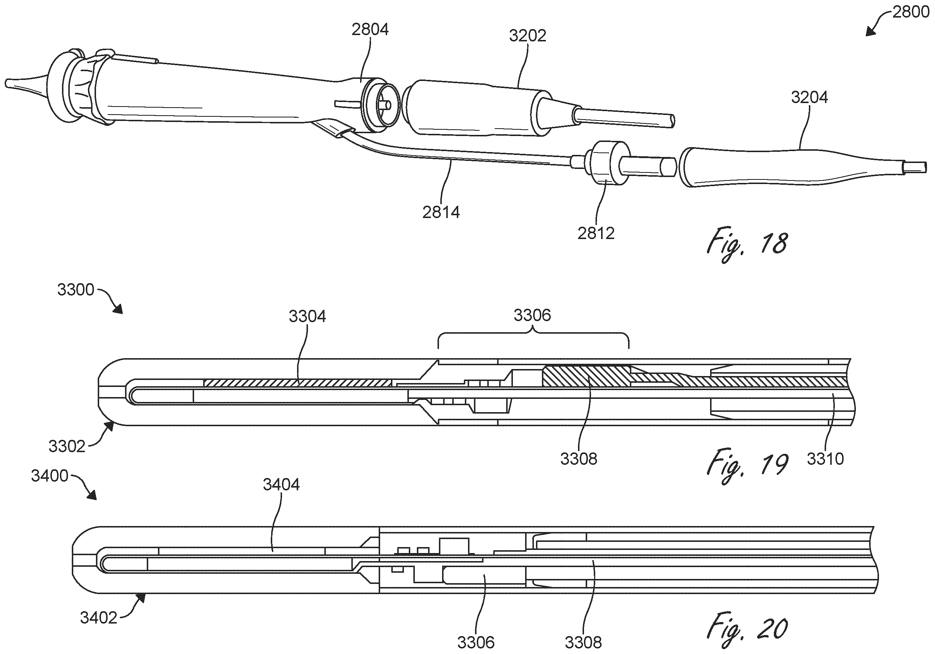

Reposing the device can involve disconnection of one or more proximal electrical contacts and moving the tool portion distally out of the distal end of the sheath portion. In this example tool portion 1212 comprises at least a tool outer sheath or member 2010, distal working end 1821 (which can include at least one ultrasound transducer), and conductor bundle 2020. The conductor bundle 2020 extends from the distal working end 1821, through the tool outer member 2010 to a proximal connector (the connector and handle mechanism are not shown in Fig. 18 for clarity). In some examples the medical tool is used for ultrasound imaging, optionally where the distal working end 1821 comprises a two-dimensional (2D) array of piezo electric components mounted on an ASIC (application specific integrated circuit).

Figure 5 illustrates a merely example proximal end of a medical device (the medical device is shown on the right), and in this example the medical device is an ultrasound probe. The proximal end 2015 of the medical device is adapted to be electrically coupled to connector cable 270, which is directly or adapted to be indirectly electrically coupled to an energy console, such as an ultrasound energy console. As illustrated in Figure 5, flexible conductor bundle 2020 extends from a distal region of the medical tool (distal region not shown) into a proximal connector 2015 within which is housed a rigid or flexible printed circuit board ("PCB") 2030. The connector bundle 2020 includes a plurality of contacts 2024 (examples of which are described below) that are attached to PCB board contacts 2031. Each individual trace from each contact 2031 is linked to individual exposed contacts 2050 on another portion, optionally more proximal, of the PCB. The individual PCB traces may also pass through other useful circuitry on the PCB. The exposed contacts 2050 are configured for a mechanical mating for electrical conduction to similar contacts 2060 on mating connector cable 2070, similar in concept to the proximal tool connector 1990 described previously, which links the tool 1204 to a user-interface console. Proximal connector 2015 can be incorporated into any of the systems, handles, steerable sheaths, medical tools, etc., herein.

"Conductor bundle" as that term is used herein may be interchangeably used with "flexible conductor bundle" unless indicated to the contrary herein.

Figures 6A and 6B illustrate an example conductor strip (also referred to herein as a flexible circuit strip) 2021 that can be included in any of the conductor bundles herein. The example in Figures 6A and 6B is an example of a conductor strip that can be included in bundle 2020 from Figures 4 and 5. The example in Figures 6A and 6B can be incorporated into any other system herein.

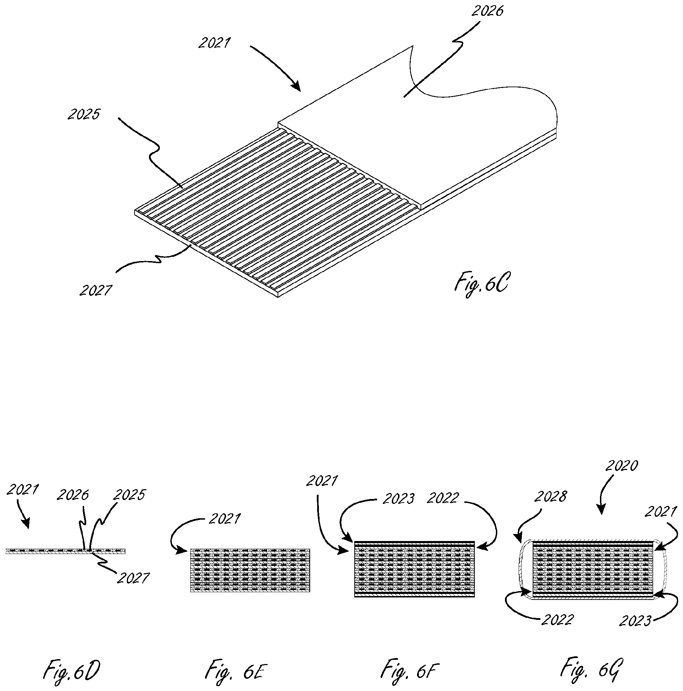

As shown in Figures 6A, 6B and 6G, conductor bundle 2020 comprises a plurality of flex circuit strips, including multi-trace strips 2021, as well as conductive strips for grounding 2022 and shielding 2023 (only a portion of which are shown). Each multi-trace strip comprises a plurality of conductive traces 2025, which can be seen clearly in Figures 6B, 6C and 6D. The number traces 2025 in Figures 6D-G is twelve, and the number of traces in Figures 6A-6C is sixteen, and they are both example as to the number of traces 2025 that can be used. Each strip 2021 can be approximately .072" wide and .0022" thick, and can optionally comprise sixteen .0022" wide x about.0007" thick conductive (e.g., copper) traces, each spaced approximately .0022" apart. The traces are disposed on an insulating substrate layer 2027, such as a polyimide substrate, and the traces can be at least partly covered by a cover layer 2026, such as a photoimageable film cover ("PIC") layer or other dry film solder mask (DFSM) or other similar material. The cover layer generally extends along most of the bundle, except at discrete locations in proximal and distal regions for electrical coupling. In other examples, the strip 2021 is approximately .055" wide and comprises twelve conductive traces (see figures 6D-G). In other examples, the strip 2021 is approximately .037" wide and comprises eight copper conductive traces. The outer strips 2022 and 2023 used for grounding and shielding may have a similar construction and dimension except they can comprise a single full width strip of copper. As optimized for a 2D piezo array, a stack of approximately seven 16-trace strips 2021 would be required (or nine 12-trace, or fourteen 8-trace), along with one each of strips 2022 and 2023 on each side of the stack of multi-trace strips. Figure 6E illustrates a portion of an example bundle 2020 with nine strips 2021 stacked together. Figure 6F illustrates a portion of the bundle that includes nine strips 2021 stacked, as well as ground strip 2022 and shield strip 2023 (only those on top are labeled). The complete bundle may optionally be held together with a, for example without limitation, about.001" wall thickness shrink tube, such as the tubing 2028 in Figure 6G. The flex circuit dimensions and number of traces discussed above are for a particular configuration of a piezo-electric array (and/or an ASIC controller thereof) and may be varied depending on how the number and size of array elements are optimized for the particular application.

The proximal end of each flex circuit strip has the conductive material (e.g., gold-plated copper) exposed over a length of approximately, for example, 3mm through removal of the cover layer 2026 at location 2024. Location 2024, and other exposed locations described herein, is generally referred to as a "contact." It is understood that when used in this context, the contact actually includes a plurality of separated conductive traces (such as shown in region location), each of which is adapted to be in electrical communication with its own corresponding conductive element. "Contact" is therefore not limited to mean only a single electrical connection between two conductive elements. While Figure 6A shows a plurality of exposed regions 2024, the example in Figure 6A will first be described herein as if there is only one exposed region (i.e., region 2024 at the proximal end). The strip 2021 can be made to create an electrical connection to matching exposed contacts 2031, shown in Figures 6A-C, for conductive traces on the PCB 2030. In some examples, sixteen individual traces, sized and spaced to match sixteen traces in the multi-trace strip 2021, would be provided within a given contact 2031. An ACF (anisotropic conductive film), soldering, conductive adhesive, mechanical connection, or any combination of these may be used to achieve a suitable electrical connection (electrical coupling) between the strip traces and the PCB contacts.

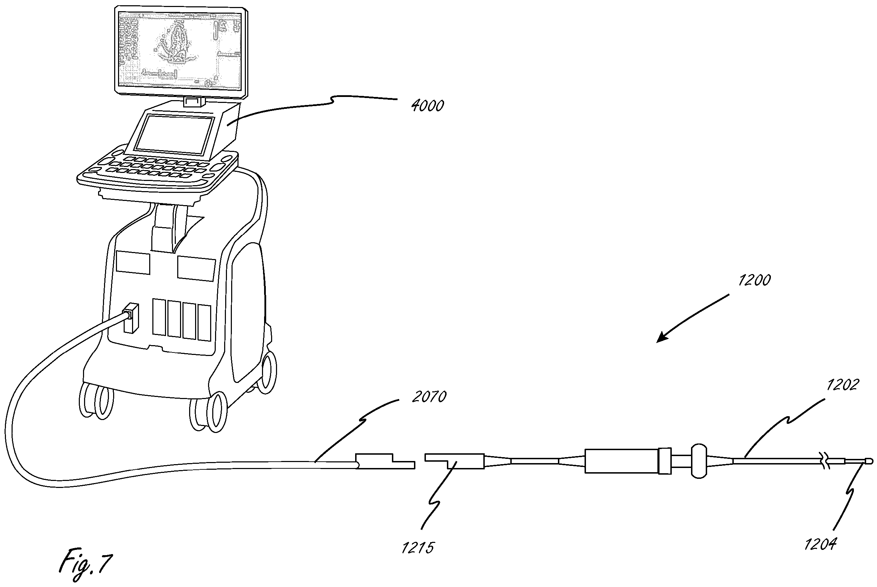

Figure 7 illustrates the integrated system 1200 of the steerable sheath 1202 and medical tool 1204 wherein the system 1200 is connected to console 4000 via the connector cable 2070. As previously described, such as for Figure 5, the tool 1204 comprises a proximal connector 2015 which forms a mating connection to cable 2070. As previously described, it is desirable to repose (e.g., reprocess and reuse) the system 1200. It is further desirable to ensure that the system is reposed only by the original manufacturer and not an unaffiliated third-party, and to ensure the device is only reused a specified number of times. To control the reposing process, a crypto-authentication chip (crypto-chip) is incorporated into the tool 1204, preferably on the PCB 2030, although other locations, such as within the steerable handle 1206, or within the tip 3000, are contemplated. The crypto-chip is programmable only by the original manufacturer who controls the authentication keys. The console 4000 to which the system 1200 is connected has a Trusted Platform Module (TPM) which also has the authentication keys. During use of the system 1200, the console 4000 is able to authenticate the system 1200 via the crypto-chip and as desired may read and write information to the chip (e.g., via an EEPROM feature). In any of the scenarios discussed, RFID chips, preferably encrypted, may be used to read and transmit data between the console, connector, and the device.

As used herein, "cleaning" can refer to any type of cleaning, such as without limitation: cleaning an interior of an outer shaft using a flushing system of cleaner and/or disinfectant and optionally mechanical scrubbing with small brushes; mechanical cleaning (e.g., wipes, brushes) an outer portion of an outer shaft and/or outer portion of a medical device shaft (e.g., ultrasound probe) with a cleaner/disinfectant, and optionally submerging the shaft in an ultrasound bath of cleaner/disinfectant for a specified period of time; and optical cleaning methods such as comprising using UV light. "Cleaning" as used here does not refer to a specific cleaning process, but rather refers to the general idea of cleaning an object.

The disclosure herein also includes methods of assembling or reassembling any of the subassemblies or assemblies herein, including any of the subassemblies within any of the handle assemblies herein. For example without limitation, the disclosure here includes methods of spooling one or more pull wires over a bearing surface in a spindle support and then around the spindle.

The methods herein also include manufacturing or constructing any of the individual components of any of the subassemblies or assemblies herein. For example, the disclosure includes methods of manufacturing handle shell components that have particular configurations (e.g., guides, walls, etc.) that can accommodate the internal parts that allow the assemblies or subassemblies herein to function as intended.

Regardless of the reference number with which they are labeled, any of the handle assemblies, medical tools, steerable sheaths, and electrical connections herein can be used together in a system in any combination with each other.

Any of the technology, including ultrasound and steering technology, in any of the following U.S. patent references may be incorporated into any of the medical tools, devices, systems, or methods of use thereof herein, the disclosures of which are incorporated by reference herein: 6100626, 6537217, 6559389, 7257051, 7297118, 7331927, 7338450, 7451650, 7451650, 7527591, 7527592, 7569015, 7621028, 7731516, 7740584, 7766833, 7783339, 7791252, 7791252, 7819802, 7824335, 7966058, 8057397, 8096951, 8207652, 8207652, 8213693, 8364242, 8428690, 8451155, 8527032, 8659212, 8721553, 8727993, 8742646, 8742646, 8776335, 8790262, 8933613, 8978216, 8989842, 9055883, 9439625, 9575165, 9639056, and 20080287783.

Any suitable disclosure above can be incorporated into any of the examples below. For example, aspects of devices, systems, and methods of manufacture and use are incorporated herein and can be incorporated into any of the examples below unless specifically indicated to the contrary.

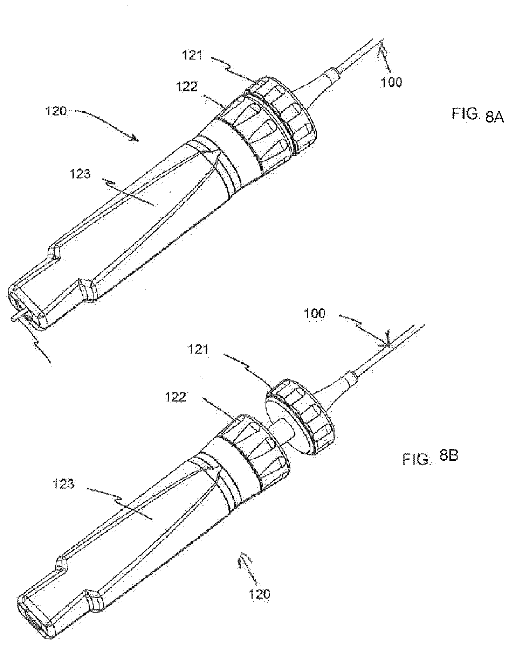

Figures 8A and 8B illustrate a merely example handle assembly that may be in operable communication with outer shaft 131 and inner shaft 132. In this example implementation, handle assembly 120 includes handle body 123 that has an outer surface that can be gripped by a user, first actuator 121, and second actuator 122. Actuator 121 can be in operable communication with outer shaft 131, and actuator 122 can be in operable communication with inner shaft 132. Actuator 121 is adapted to be both rotated and moved axially relative to handle body 123 (and relative to second actuator 122). This allows actuator 121 to cause axial movement of the medical tool 103 and rotation of the medical tool 103 relative to a distal end of inner shaft 132. Second actuator 122 is adapted to be actuated (e.g., rotated in this example) relative to handle body 123 to cause deflection of the inner shaft 132. For example, the handle assembly can have internal components that interface with proximal ends of pull wires such that actuation of actuator 122 tensions one or more pull wires to cause deflection of the inner shaft. In this example actuator 121 is distal to actuator 122, but in other designs their relative positions could be reversed. Figure 8B shows handle assembly 120 after actuator 121 has been advanced distally relative to its position in figure 8A. This distal advancement causes outer shaft 131 to be advanced distally, and thus causes the medical tool to be advanced distally. Actuator 121 can similarly be retracted proximally relative to its position in figure 8B. Tensioning members described further below are referenced with respect to actuator 121 being retracted proximally.

In other designs, actuator 121 could be in operable communication with an inner shaft and actuator 122 may be in operable communication with an outer shaft.

As described herein, the outer shaft can be moved axially relative to the inner deflectable shaft. The outer shaft may be constructed with sections of materials that vary in stiffness (e.g., durometer) along the length of at least a portion of the outer shaft. For example, a first portion that is distal to a second portion can have a lower durometer than the second portion. Because the outer shaft can be moved axially relative to the deflectable inner shaft, and because the stiffness of the outer shaft can vary along its length, the deflection, including the degree (or amount), of the overall device can be selectively controlled by controlling the axial position of the outer shaft (relative to the inner shaft). Axial movement of the outer shaft can thus selectively control deflection of the device. For example, a user (e.g., physician) can change or control where the bend occurs along the length of the device (measured from the distal end) by axially moving the outer shaft relative to the inner shaft. Additionally, for example, sections of varying stiffness in the outer shaft can allow for more or less deflection depending on the relative position of the outer shaft relative to the deflectable inner shaft. For example, deflecting the inner shaft at a region where the outer shaft has a relatively higher stiffness can result in less deflection than when the inner shaft is deflected at a region where the outer shaft has less stiffness. Although reference is made to distinctions in characteristics and control of the inner and outer shafts, such control of the inner shaft and outer shaft may be reversed, wherein the inner shaft is configured for rotation and the outer shaft is configured for deflection.

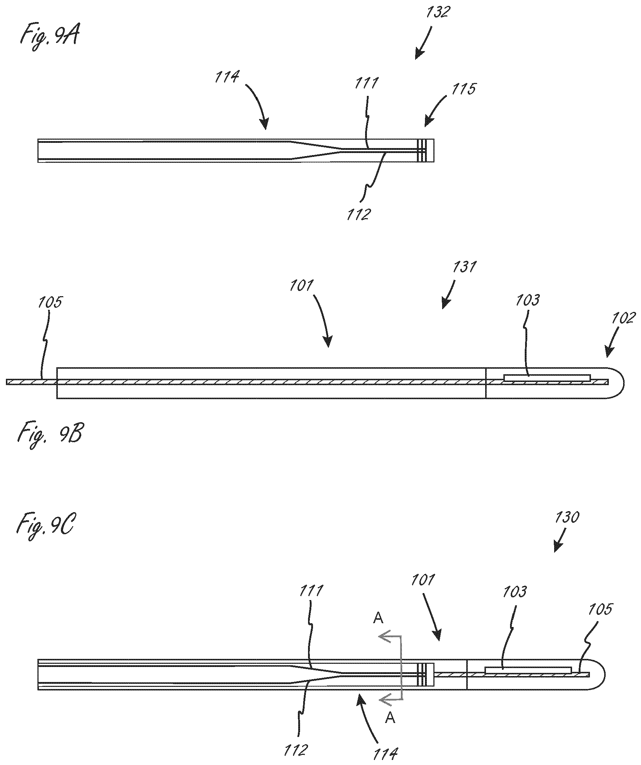

Figure 9C shows example apparatus medical apparatus 130, which includes elongate inner shaft 132 (see figure 9A) and elongate outer shaft 131 (see figure 9B). Medical apparatus 130 may also be referred to herein as a "catheter," or other medical device that includes at least one elongate shaft.

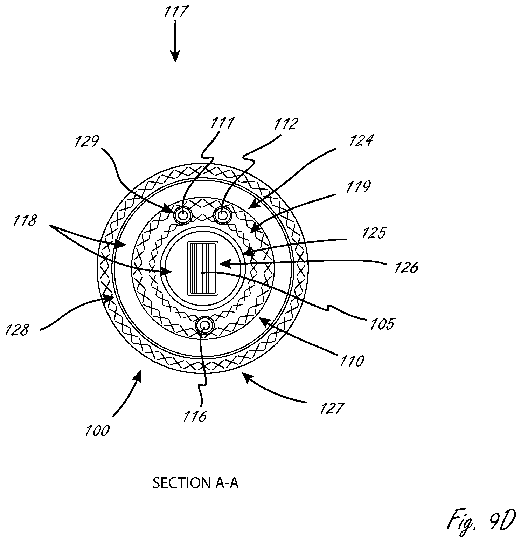

Figure 9D illustrates Section A-A shown in the assembly in figure 9C, which is a section in the deflectable section of the device. Parts from figures 9A-9C are similarly labeled. As can be seen in figure 9D, pull wires 111 and 112 are very near to one another and about 180 degrees away from straightening pull wire 116.

As is also shown in figure 9D, elongate inner shaft 132 includes two layers of braided material 119, and the pull wires are, at least at the location of this section, essentially sandwiched between the two layers of braided material. Annular spaces 118 allow freedom of movement and space for optional lubricant. Inner shaft 132 may be made from, for example without limitation, a polymeric material such as Pebax, optionally with a lubricious additive. Inner shaft 132 may include liner 125, such as a PTFE liner. The flexible cable bundle 105 may be surrounded by one or more layers of insulation 126, such as PTFE insulation. Outer shaft 131 may comprise a polymeric material 127, such as Pebax. Outer shaft 131 may also include a radially inner liner 128, such as a PTFE liner. Any of the pullwires (e.g., 111, 112, 116) may be disposed in a lumen with a liner, such as PTFE liner 129.

Medical apparatus 130 (or either of elongate shaft 132 and elongate shaft 131, individually) can be in operable communication with any of the handle assemblies herein, including handle assembly 120 shown in figures 8A and 8B.

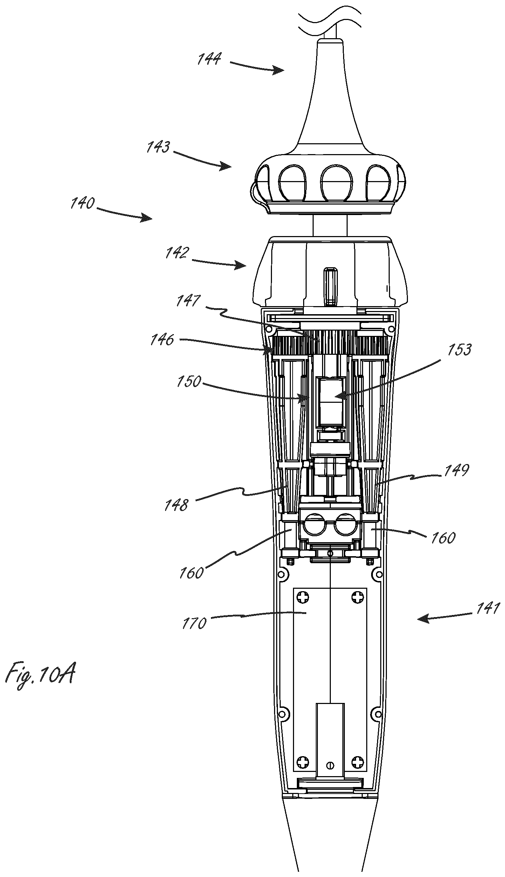

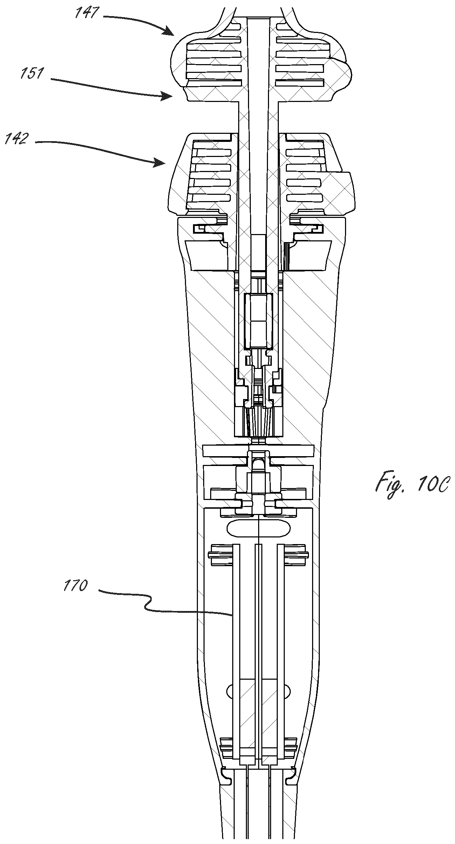

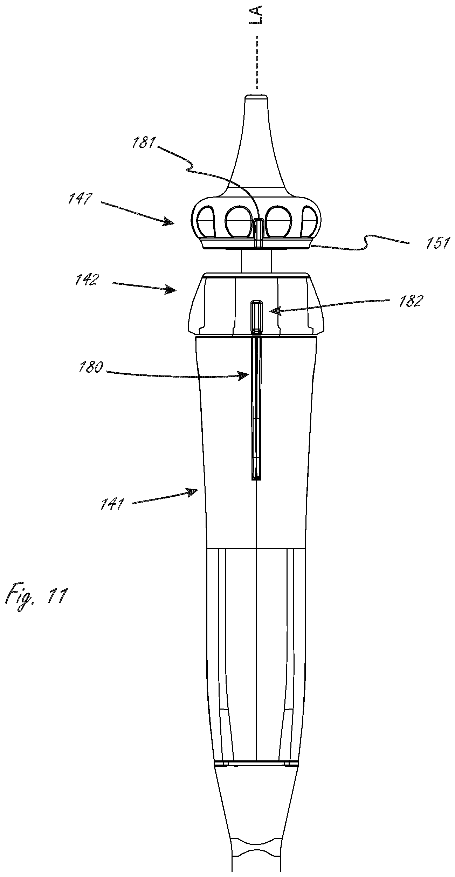

Figures 10A-10C and figure 11 illustrate an additional example handle assembly that can be in operable communication with any of the medical devices, including ultrasound probes, herein. For example, the example handle assembly shown in figures 10A-10C can be coupled to (directly or indirectly) and in operable communication with medical apparatus 130 shown in figures 9A-9D. In a particular example, both elongate outer shaft 131 and elongate inner shaft 132 are coupled to and in operable communication with the handle assembly shown in figures 10A-10C.

The handle assembly in figures 10A-10C and figure 11 has some similarities to the handle assemblies, the individual components, and subassemblies that are shown in figures 8A and 8BB. Unless indicated to the contrary, concepts, features and methods of use from figures 8A and 8B that can be incorporated into the handle assembly in figures 10A-C are hereby incorporated by reference for all purposes into the disclosure of the handle assembly shown in, and described with respect to, figures 10A-C. Similarly, concepts, features and methods of use that are shown in, and described with respect to, figures 10A-C that can be incorporated into other handle assemblies herein are hereby incorporated by reference for all purposes into the disclosure of any of the handle assemblies set forth herein.

Figure 10A is side view of handle assembly 140 with a portion of handle body 141 removed so that some internal components of the handle assembly can be seen. Handle assembly 140 includes first actuator 143 and second actuator 142, and in this example first actuator 143 is distal to second actuator 142. First actuator 143 can be both moved axially and rotated relative to the handle body and relative to a second actuator (in this example actuator 142). First actuator 143 is in operable communication with an outer elongate body, such as outer shaft 131 (*see* figure 9B). Axial movement of actuator 143 (distally or proximally) causes axial movement of the outer shaft 131, while rotation of actuator 143 causes rotation of the outer shaft. Second actuator 142 is in operable communication with an inner shaft, such as inner shaft 132 (figure 9A). Actuation of second actuator 142, in this example rotation, causes deflection of the inner shaft. In this example a rotatable and axially movable actuator (i.e., first actuator 143) is in operable communication with an outer shaft. Although reference is made that actuation of second actuator 142 may cause deflection of the inner shaft, alternatively, actuation of second actuator 142 may cause rotation of the inner shaft. Likewise, actuation of actuator 143 may alternatively cause deflection of the outer shaft.

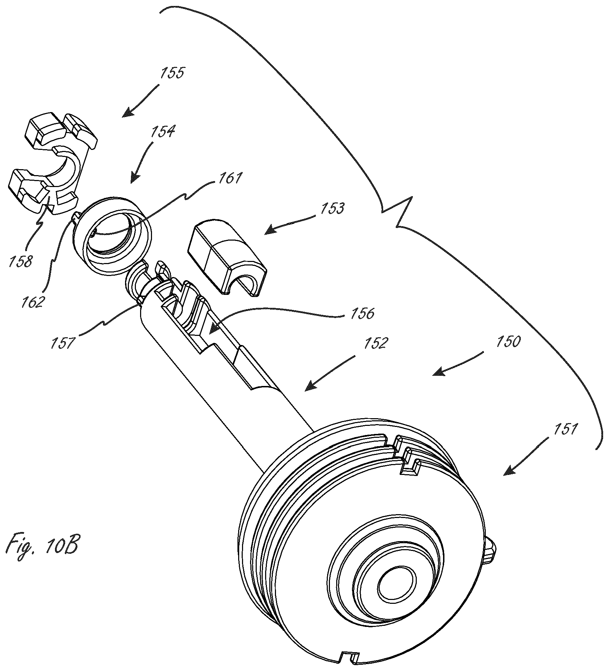

First actuator 143 is coupled to elongate outer shaft movement assembly 150 shown in the exploded view in figure 10B, such that movement of first actuator 143 causes movement of assembly 150. Elongate outer shaft movement assembly 150 is similarly coupled to the elongate outer shaft so that movement of first actuator also causes movement of the elongate outer shaft. In this example, the outer elongate shaft is attached to removable part 153 after it is inserted into channel 156. Removable part 153 and channel 156 are configured so that removable part 153 is constrained by at least one inner surface of channel 156 when it is inserted therein. Elongate outer shaft movement assembly 150 also includes a distal head portion 151 that is secured to first actuator. Elongate outer shaft movement assembly 150 also includes a rotation limiting mechanism similar to that which is described herein, which limits the rotation of first actuator 143, and thereby limits the rotation of the outer elongate shaft. Any of the disclosure above related to rotation limiting subassemblies, functionality, and use, is incorporated into this example for all purposes and may be incorporated into this and similar designs. During rotation, part 157 (see fig. 10B) interacts with part 161, and part 162 interacts with part 158. The physical interactions of these two sets of parts limits rotation to the desired rotation limit, e.g., such as limiting rotation up to 630 degrees of rotation of the outer body (in other examples the allowed rotation could be more than 630 degrees, such as up to and including 720 degrees).

If it is desired to clean the outer shaft, for example after use, removable part 153 can be detached from the outer shaft to allow the outer shaft to be removed from the handle assembly and cleaned, before being reinserted and reattached to removable part 153 or a new removable part if part 153 is damaged or broken.

Handle assembly 140 also includes inner shaft deflection assembly 146, which is in operable communication with second actuator 142. Inner shaft deflection assembly 146 includes central gear 147 adapted and configured to rotate when second actuator 142 is rotated. Central gear 147 interfaces first spindle 148 and second spindle 149 via a geared interface, such that rotation of central gear 147 causes rotation of the spindles in the opposite direction. The inner shaft deflection assembly 146, including the spindles, extends further proximally than the elongate outer body movement assembly 150. The inner shaft extends through the outer shaft and extends further proximally than the outer shaft within handle assembly 150. This allows one or more pullwires that are part of the inner shaft to extend radially outward and interface with reels 160.

The lack of interaction between elongate outer shaft movement assembly 150 and elongate inner shaft movement assembly 146 allows for the inner and outer elongate shaft to be independently controlled by first actuator 143 and second actuator 142.

Handle assembly 140 also includes printed circuit board ("PCB") 170 disposed within handle body 141, the PCB being in electrical communication with a cable bundle, such as flexible cable bundle 105 in figure 53, or any of the cable bundles herein that are in communication with the medical tool, such as an ultrasound transducer.

Handle assembly 140 also includes a rotation indicator 180 that can be used to show a user the extent to which at least one of the first actuator and the second actuator are rotated relative to a home, or neutral position. First actuator 143 can include a rotation indicator 181 that is aligned along an axis with rotation indicator 180 when first actuator 143 is in a neutral position, as shown in figure 11. When first actuator 143 is rotated, rotation indicator 181 is rotated relative to the axis along which rotation indicator 180 extends, which enables the user to visually understand that first actuator 143, and thus the outer shaft, is rotated to some extend relative to the neutral position. Similarly, second actuator 142 can also have rotation indicator 182 that is aligned along an axis with rotation indicator 180 when second actuator 142 is in a neutral position, as shown in figure 11. When second actuator 143 is rotated, rotation indicator 182 is rotated relative to the axis along which rotation indicator 180 extends, which enables the user to visually understand that second actuator 143, and thus the inner shaft, is deflected to some extent relative to its neutral position.

In some alternative examples, the handle assembly can include one or more sensors to track how much rotation has occurred for the outer shaft, or how much deflection has occurred in the inner shaft. In some examples the handle assembly can include an encoder for each actuator.

In any of the examples herein that include an outer shaft and an inner shaft, the device can include one or more lubricants between the inner and outer shafts to make it easier to move the inner and outer shafts relative to one another by reducing friction between the two. If the medical device needs to be cleaned for reuse, additional lubricant can be added between the inner and outer shafts after the cleaning process.

In some examples herein the medical device may include a flexible member, such as a flexible conductor bundle (which may be referred to herein as a conductor bundle, flex bundle or other similar derivative thereof), coupled to and extending from a distal region (e.g., probe tip) towards a proximal region of the medical device (see, for example, conductor bundle 2020 shown in example figures 4-6G; or bundle 105 from figure 9B). A probe tip may include an ultrasound transducer in electrical communication with a flexible conductor bundle. In some examples herein (e.g., figures 9A-11), the probe tip and conductor bundle can be axially displaced (proximally and/or distally) by actuation of a handle actuator (e.g., actuator 143 as shown in figure 10A). In some instances, the conductor bundle is disposed within an elongate member (e.g., steerable inner elongate body 132; or elongate member 131) and moves axially relative thereto when the probe tip is advanced distally or retracted proximally. When the distal region (e.g., ultrasound probe) and conductor bundle are retracted proximally (after being advanced distally), the conductor bundle may tend to fold up, bunch up, or otherwise bend, near or adjacent to its distal end due to friction between the bundle and, for example, the elongate member (e.g., steerable inner shaft 132) in which the conductor bundle is disposed. Bunching may occur if the medical device is in a straight configuration as well as if the medical device has some degree of bend (e.g., after being deflected from a straight or linear configuration).

To reduce the degree of, or even prevent completely, a tendency to bunch up or bend, any of the medical devices herein may include a structural tensioning member that is adapted and configured to apply or maintain tension on the flexible member, such as a flexible conductor bundle, at a location that is proximal to where the conductor bundle is coupled to the distal medical tool. By tensioning the flexible member, folding or bunching up of the flexible member can be minimized or even prevented. When used in this context, the "tensioning" member is adapted and configured to reduce distal region(s) of the flexible conductor from bunching (compared to a device without a tensioning member) by proximally moving at least a distal portion of the flexible conductor bundle as the distal probe is retracted proximally. In some examples, the structural tensioning member (e.g., a tensioning bar) may be physically secured to the flexible conductor bundle (e.g., direct or indirect attachment). In general, the tensioning members herein are in operable communication with the flexible members (e.g., a flex conductor bundle) such that movement or actuation of the tensioning member applies some force to the flexible member, and may cause movement (e.g., proximal) of the flexible member. In some example examples the structural tensioning member may be disposed in or carried by the handle assembly of the medical device. Structural tensioning member in this context may be a single component or an assembly of separate components.

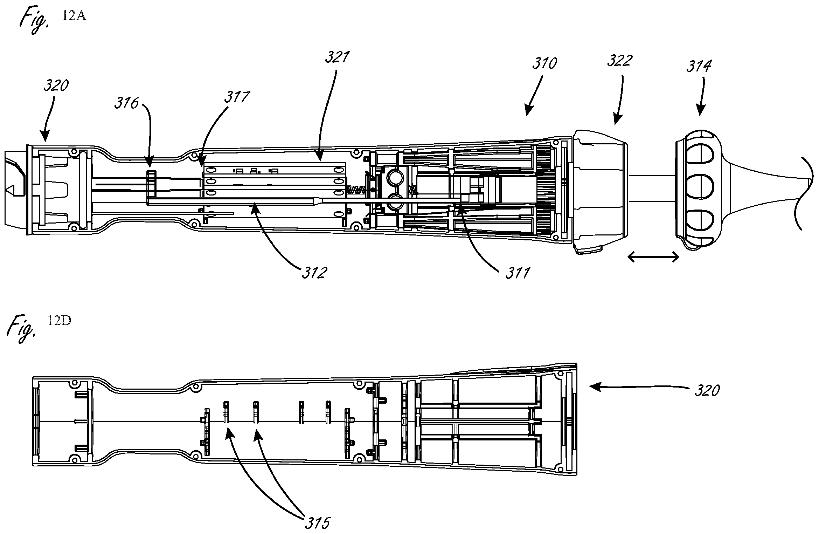

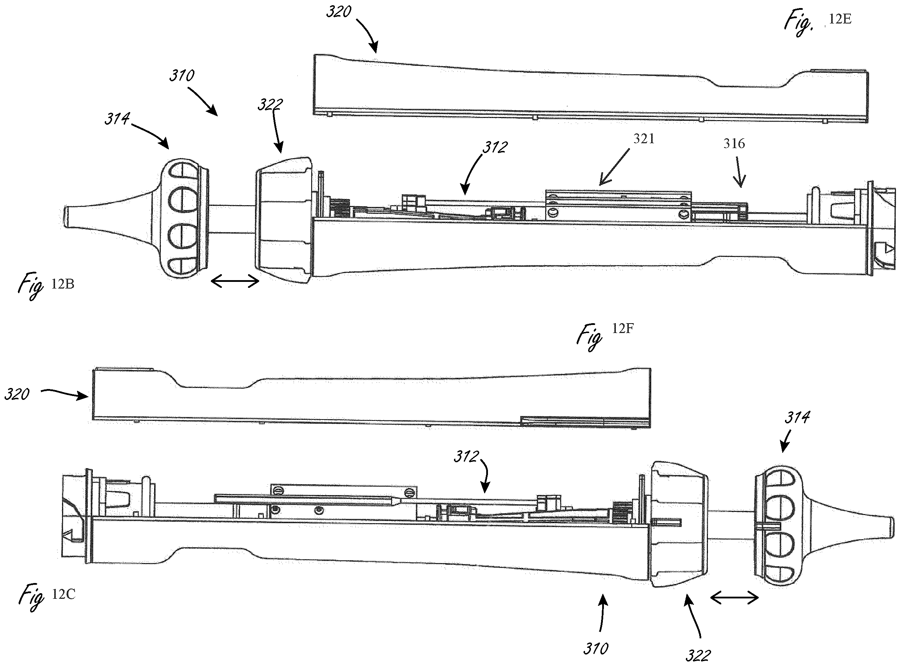

Figures 12A-12F illustrate a handle assembly portion of an example medical device, which may be incorporated into any suitable medical device herein. For example, the handle assembly in Figures 12A-12F may be part of a medical device that includes a medical tool (e.g., an ultrasound imaging probe) at its distal end or near its distal end, examples of which are described herein. Any other example or feature herein is incorporated by reference into the example handle assembly shown in figures 12A-12F.

Example handle assembly 310 includes first actuator 314 and second actuator 322, where first actuator 314 is distal to proximal actuator 322. First actuator 314 is adapted and configured to be moved axially (distally and proximally) relative to second actuator 322 (and optionally also rotatable relative thereto), and may be in operable communication with an elongate body (e.g., 131 or 132) that may include a medical tool (e.g., 103) in a distal region. Handle assembly 310 (including actuators) may incorporate any of the relevant disclosure from any other handle assembly herein. The medical device of which handle assembly 310 is a part also includes a flexible member (e.g., flexible conductor bundle 105 in figure 9B) securely coupled to (directly or indirectly) the medical tool at a first distal location (e.g., as shown in figures 9B and 9C where medical tool 103 is secured to flexible member 105) and extends proximally from the medical tool towards handle assembly 310. The flexible member may be a flexible conductor bundle and can extend into handle assembly 310, as shown. A portion of the flexible member is disposed within an outer surface of the elongate body (e.g., within 131 and/or 132). The medical device also includes tensioning member that is secured to the flexible member at a second location 316, which is proximal to the first location ("first location" in this context may be referred to as a first distal location or derivative thereof). Figure 12A illustrates example tensioning member 312, while reference number 312 in figure 12A also points to an optional elongate rigid member (in this example the elongate rigid member is linear and extending axially) of the tensioning member. Tensioning member 312 is adapted and configured to tension the flexible member as the medical tool is retracted proximally. This may be referred herein as applying a tensile force to the flexible member, or tensioning the flexible member, or maintaining tension in the flexible member. In this example example, tensioning member 312 is adapted and configured to tension the flexible member as the medical tool is retracted proximally, which in this example occurs when first actuator 314 is retracted proximally from its position shown in figure 12A-C. The tensioning members herein can apply tension when the medical device is in a straight configuration and when the medical device is in a non-straight (linear) configuration, such as when the device may be deflected or bent.

In this example, tensioning member 312 (which may include the tensioning bar shown in figure 12A) is secured to (e.g., attached directly) the flexible conductor bundle at location 316, the location of which is inside the handle assembly in this example. A tensioning member may alternatively be secured to the flexible member at a location that is not within a handle, such as outside of the handle, such as inside one or both of outer and inner shafts. In this example the tensioning member is also axially secured relative to first actuator 314, such that axial movement of first actuator 314 causes axial movement of tensioning member 312 (which may be in a 1:1 movement ratio). Because tensioning member 312 is also secured to the flexible member (e.g., at location 316), axially movement of tensioning member 312 also causes axial movement of the flexible member at location 316. By securing tensioning member 312 to the flexible member, the flexible member is tensioned distal to where the tensioning member is secured to the flexible member when first actuator 314 is retracted proximally, which prevents the flexible member from folding or bunching up at its distal region near or adjacent to the medical tool (or at least reduces the extent of folding/bunching up compared to a device without a tensioning member).

The flexible member may include a flexible conductor bundle, such as any of the flexible conductor bundles herein. In figures 12A-F, the tensioning member comprises a rigid elongate member (generally referred to as 312 in figure 12A) with a fixed length. The rigid tensioning member as shown has a general longitudinal axis, which in this example is parallel with a longitudinal axis of the medical device and/or a longitudinal axis of the handle assembly. The rigid tensioning member may be made of a variety of materials, such as a rigid plastic member.

The tensioning members herein can ensure that the distance traveled by the medical tool is the same as the distance traveled by any point on the flexible member between the first and second locations. The tensioning members herein can ensure that the distance traveled by the medical tool is the same as the distance traveled by the location where the tensioning member is secured to the flexible member (e.g., location 316 in figure 12A).

The secured relationship between the tensioning member and the flexible member maintains the flexible member in a substantially flat or straight configuration between the first and second locations as the medical tool is retracted proximally (when the medical device is a straight configuration). A flat configuration in this context can include examples in which the flexible member may also be twisted (i.e., the flexible member can be flat and still be twisted, but not bunched/folded). A flattened configuration as used herein indicates a lack of a fold or bunching of the flexible member.

Medical devices in which a tensioning member is incorporated may also be steerable or deflectable. The tensioning members herein can be adapted and configured to apply a tensile force to the flexible member even if the medical device, including the flexible member, are in non-straight (e.g., deflected, steered, bent) configurations. When this disclosure refers to maintaining a substantially flat configuration in the flexible member, it refers to instances where the medical device may be in a straight configuration, which need not be the case, such as when a medical device has been steered, bent, or deflected.

The secured relationship between the tensioning members and the flexible members herein prevents the flexible member from forming a fold (i.e., bending, or bunching up) between the first and second locations as the medical tool is retracted proximally. Folding, bending, and bunching-up in this context includes a first region of the flexible member axially overlapping with a second region of the flexible member, and also includes general bending and bunching of the flexible member, such as regions of the flexible member that are not flat and, for example, form a bend, curved region, and/or meander back and forth.

The flexible member may have flat top and bottom surfaces (e.g., a conductor bundle with one or more flat surfaces), and optionally the tensioning member may be secured to at least one of the top and bottom surfaces. For example, figures 6A-6G illustrate a flexible member with flat or generally flat first and second surface (e.g., top and bottom surfaces), and a tensioning member may be secured to one or both of the flat or generally flat surfaces (e.g., such as at location 316). They may be secured using a variety of techniques, such as using adhesive, welding, or other bonding techniques.

The tensioning member may be in operative communication (directly or indirectly) with a handle actuator such that axial movement of the actuator axially moves the tensioning member. The handle actuator may be further adapted and configured to be rotated (e.g., actuator 314) to cause rotation of the medical tool, and optionally wherein rotation of the actuator does not cause rotation of the tensioning member. The tensioning member can thus be adapted to be moved axially when the actuator is moved axially, but not to rotate when the actuator is rotated. This can be accomplished by the manner in which the tensioning member is operatively in communication (directly or indirectly) with the actuator.

The medical device may include an inner elongate body (e.g., 132) including a lumen in which at least a portion of the flexible member is disposed. An inner elongate body may be independently steerable, such as with a separate independently actuatable handle actuator (e.g., actuator 322). Aspects of other examples herein in which the medical device includes an inner member and outer member, the inner member independently controllable (e.g., axially and rotationally) are fully incorporated in any of the examples herein.

The flexible member may be coupled to a printed circuit board (e.g., 321 "boards" as shown in figure 12A) in the handle assembly. The tensioning member may be coupled to a flexible member proximal to a printed circuit board. In alternative examples the tensioning member may be coupled to a flexible member distal to a printed circuit board.

Figures 12A-12F is an example of a portion of a medical device that includes an elongate outer body (e.g., 131) including a probe tip in a distal region of the elongate body; a flexible conductor bundle (e.g., 105) securely coupled to the probe tip at a first location (shown in figure figures 9B and 9C) and extending proximally from the medical tool and into a handle assembly, the flexible member disposed within an outer surface of the elongate body; an inner elongate body (e.g., 131), at least a portion of which is disposed within the elongate outer body, the inner elongate body optionally steerable, wherein at least a portion of the flexible conductor bundle is disposed within the inner elongate body and configured to be axially movable relative to the inner elongate body; a tensioning member secured to the flexible member at a second location in the handle assembly, the tensioning member adapted and configured to apply tension on the flexible member as the probe tip is retracted proximally. Probe tips as used herein may include one or more ultrasound transducers.

Figures 12E-F illustrate a half of a handle outer shell 320, two of which form a portion of the outer surfaces of the handle assembly 310. Handle shells 320 include radially inwardly extending features 315 that are adapted to interface with a control the movement of the tensioning member 312. Features 315 can include guides that are configured to interface with the tensioning member at one or more locations and help stabilize the tensioning member.

Any other handle assembly component in any other example herein that can be suitably integrated into handle assembly 310 is incorporated by reference herein.

In any of the examples and claims herein, the phrase "tensioning member" may be replaced with "straightening member," "flattening member," or a derivative thereof. As described herein, a straightening member or flattening member refers to maintaining a substantially straight or flattened configuration in the flexible member when the medical device is in a straightened configuration, and does not require that the device always has a straightened configuration. Thus, even if the flexible member is not necessarily being placed under tension, it may still be maintained in a straightened (i.e., not folded configuration) along at least a portion of its length due to a straightening member. Member 312 in figure 12A, for example, is an example of a straightening member, even if it also functions as a tensioning member. This applies to all tensioning members described, shown, and claimed herein. In some examples herein, the "member" can be a straightening member (or flattening member) and can also function as a tensioning member. Additionally, the phrase "tensioning member" herein may be replaced with "fold-prevention member," "bend-prevention member," "bunching-prevention member," or a derivative thereof.

The disclosure above describes that in some examples the flexible member, such as conductor bundle 2010, may be twisted along a portion of its length. For example, a conductor bundle may be twisted to provide a more balanced cross-section along a portion of the length of the medical device. A conductor bundle may be twisted only in a portion of the medical device that will experience deflection.

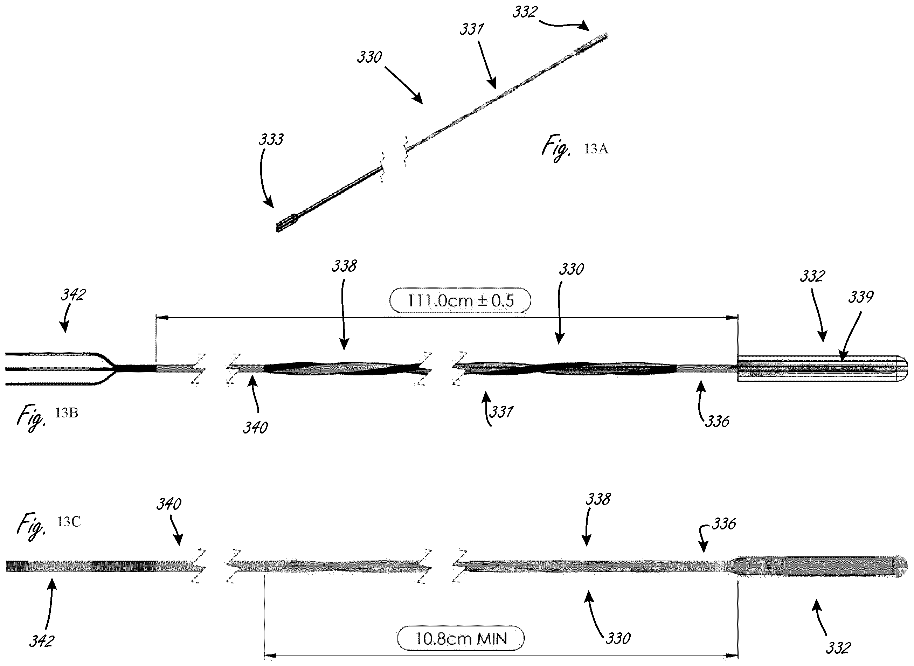

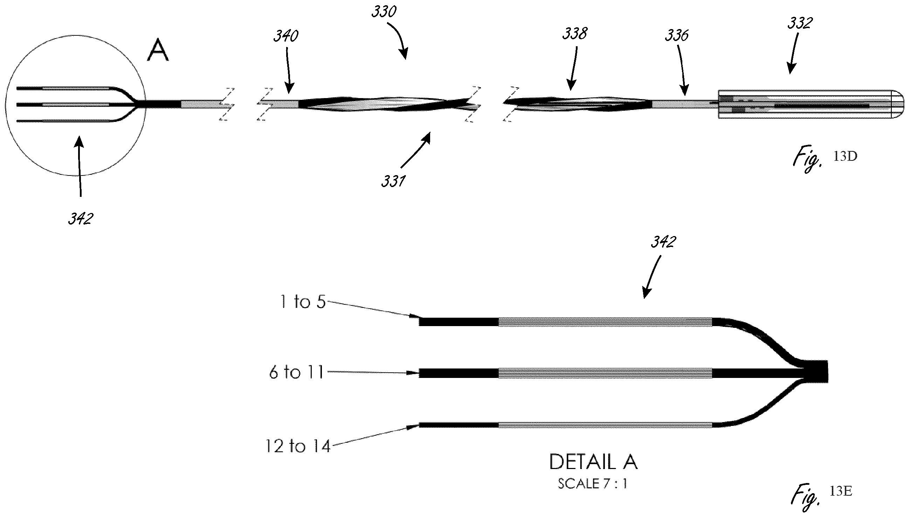

Figures 13A-13E illustrate a portion of an example medical device that includes a twisted flexible member, such as a flexible conductor bundle. The example in figures 13A-E may be incorporated with any other suitable feature and/or medical device described herein.

The portion 330 of the medical device shown in figures 13A-E includes a medical tool 332 at a distal region, a flexible member 331 coupled thereto and extending proximally therefrom, and a proximal end region 333 comprising a plurality of electrical connectors. Flexible member 331 may be a flexible conductor bundle, such as any of the bundles herein. Medical tool 332 may include an ultrasound imaging transducer 339. Flexible conductor bundle 331 has a region 338 in which the conductor bundle is twisted, the twisted region 338 having a distal end and a proximal end. Flexible conductor bundle 331 also includes a region 336 distal to twisted region 334 that is not twisted, and a region 340 proximal to twisted region 336 that is not twisted. Medical tool 332 may be coupled to an outer shaft, such as outer shaft 131 shown in figure 9B.

The length of twisted region 338 from its distal end to its proximal end can vary, and in some examples is from 5-15 cm, such as from 8-15 cm, such as 11 cm. The length over which a complete turn is formed can vary well, such as from 1-5 cm, such as 3 cm.

The number of twists over the length of the twisted region can vary as well, such as, for example without limitation, 7-9 full twists.

An example manner in which to form the twisted region of the flexible member (e.g., flexible conductor bundle) is to couple the flexible member to the medical tool at the distal end (e.g., probe tip). A thin section of PET heat shrink may then be advanced over the flexible conductor bundle. A portion of the device can be held in place while another portion is twisted to the desired number of turns to form the twisted region. While the twisted configuration is maintained, the PET can be heat shrunk over the twisted bundle region. Additional layers of PET may be subsequently added. An elongate member (e.g., shaft 131) may then be placed over the bundle, including the twisted region, and the elongate member can be bonded to the medical tool.

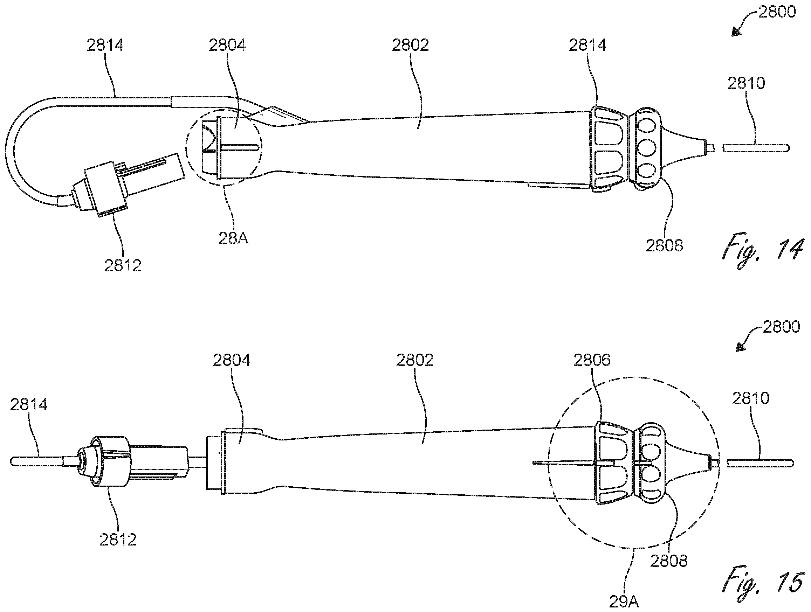

A Referring to FIGS. 14-18, a system 2800 includes a handle assembly 2802 and steering and medical device portion 2810 (e.g., shown as an outer sheath similar to sheath 1208 (FIG. 4), which may further enclose an inner shaft and tool. The system is adapted so that handle assembly 2802 can be actuated to cause steering of a steerable portion of the steering and medical device portion 2810, and optionally can be further actuated to cause movement of a medical device coupled thereto. As an illustrative example, the handle assembly 2802 includes a first actuator 2806 and a second actuator 2808. One or more of the first actuator 2806 or the second actuator 2808 is adapted (e.g., configured) to be actuated (in this example rotated) relative to a handle body of the handle assembly 2802 to cause the steering of steerable portion 2810, such as steering an outer sheath. Steering may include rotating or deflecting. As an example, one of the actuators 2806, 2808 controls rotation, while another of the actuators 2806, 2808 controls deflection. As a further example, one of the actuators 2806, 2808 controls rotation of one or more of the inner shaft or outer shaft, while another of the actuators 2806, 2808 controls deflection of one or more of the inner shaft or the outer shaft.

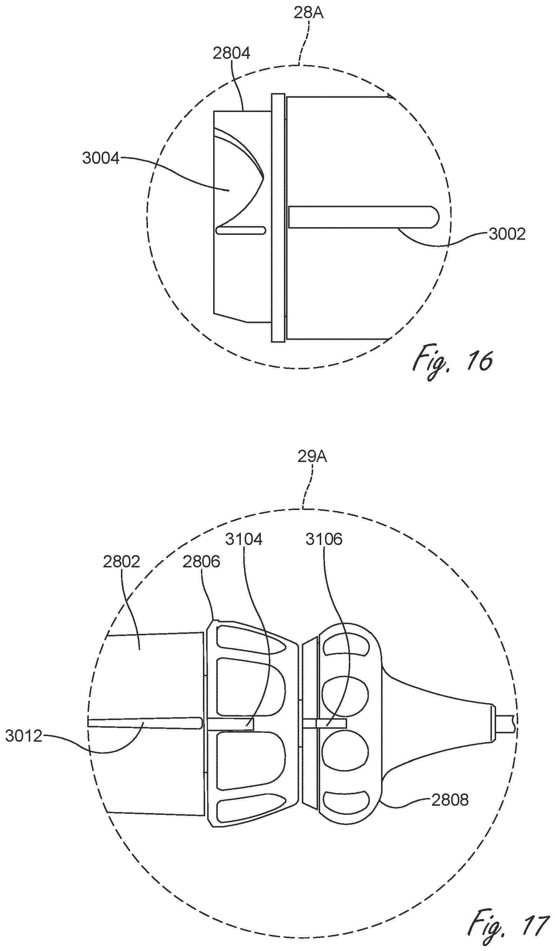

As more clearly shown in FIG. 17, a neutral position marker 3102 may be disposed on a body of the handle assembly 2802 to indicate (e.g., visually, tactilely, etc.) a fixed reference point. One or more of the actuators 2806, 2808 may further include a marker 3104, 3106 to indicate alignment or position of the actuators 2806, 2808 relative to the neutral position marker 3102. As an example, the marker 3104 may indicate a deflection of the shaft relative to the neutral position marker 3102 and the marker 3106 may indicate a rotation of a component (e.g., transducer face disposed in the sheath 2810) relative to the neutral position marker 3102, or vice versa. The markers 3104, 3106 may indicate other positions. Additionally, or alternatively, mechanical registration mechanisms, such as detents, may be used to provide tactile feedback to a user to indicate certain positions of the actuators 2806, 2808, such as when the actuators 2806, 2808 are in the neutral position.

The handle assembly 2802 may include any other handle component or functionality described in any of the other handles herein. For example, a receptacle 2804 may be disposed at a proximal end of the handle assembly 2802 and adapted to receive a plug such as an umbilical plug. As more clearly shown in FIG. 16, the receptacle 2804 may comprise visual and/or tactile orientation markers for registration and alignment of an umbilical plug and the receptacle 2804.

A navigation connector 2812 may be coupled to the handle assembly 2802 via a navigation conduit 2814 (e.g., navigation cable sleeve). The navigation connector 2812 may be configured to interface with a navigation system such that the system 2800 may communicate with the navigation system. As an example, the navigation system may provide tracking and navigation of a portion of the system (e.g., the medical device) while in use. As shown, for example, the navigation conduit 2814 may couple to a body of the handle assembly 2802 to provide access for one or more cables to pass from the navigation connector 2812 to components within the housing of the handle assembly 2802. As more clearly shown in FIG. 18, the navigation connector 2812 may be configured to interface with a navigation system connector 3204 (e.g., receptacle), which may be proprietary to the navigation system manufacturer. The receptacle 2804 may be configured to interface with a controller interface 3202. Other configurations may be used.

FIGS. 19-24 illustrate an example distal region of a steerable system that includes a medical tool. As an illustrative example, FIG. 19 shows the medical tool may comprise a steerable tip 3300 disposed at the distal end of a sheath. The steerable tip 3300 may include a transducer 3304 (e.g., ultrasound transducer) at least partially enclosed by a material 3302 (e.g., polymer). The tip 3300 may comprise an electronic module 3306, which may include sensors, control boards, thermistors, and the like. As an example, a TAS sensor 3308 may be disposed in communication with the module 3306. As a further example, a sensor cable bundle 3310 may be disposed to provide electrical communication to one or more components coupled to the module 3306.

As an illustrative example, FIG. 20 shows the medical tool may comprise a steerable tip 3400 disposed at the distal end of a sheath. The steerable tip 3400 may include a transducer 3404 (e.g., ultrasound transducer, folded transducer) at least partially enclosed by a material 3402 (e.g., polymer). The tip 3400 may include sensors, control boards, thermistors, and the like. As an example, a TAS sensor 3406 may be disposed in in a configuration that is closer to the distal end of the tip 3400, when compared to the tip 3300 configuration in FIG. 19. By shifting the sensor 3406 the proportion of the tip 3400 that may be deflected is increased.

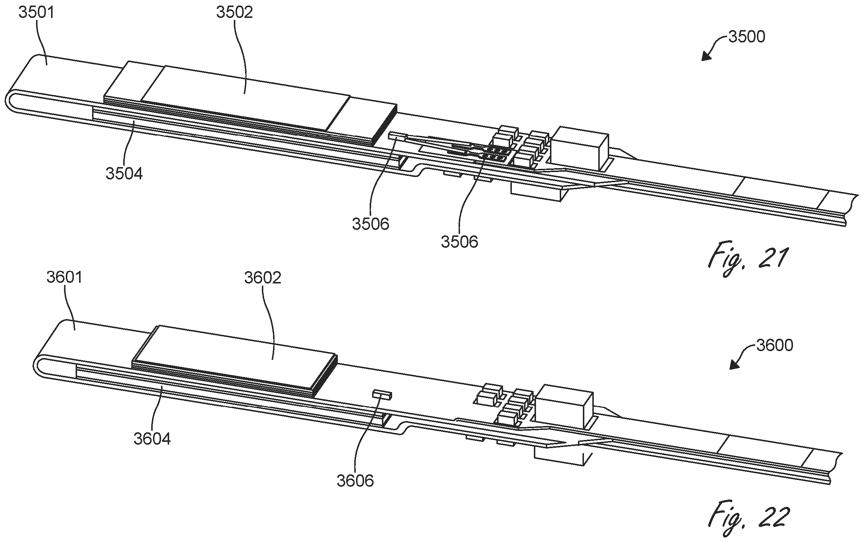

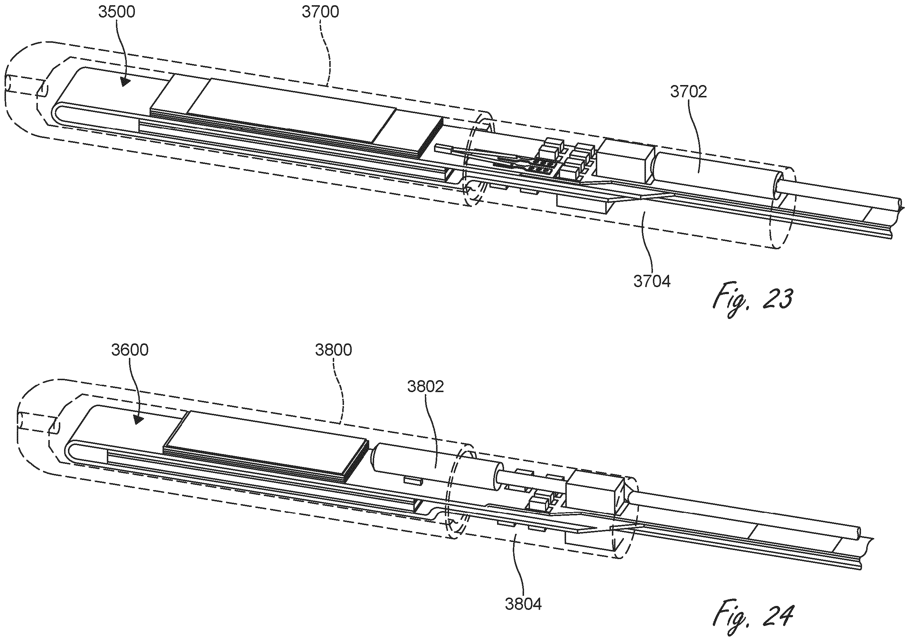

As an illustrative example, FIG. 21 shows the medical tool may comprise a steerable tip 3500 disposed at the distal end of a sheath. The steerable tip 3500 may include a transducer 3502 (e.g., ultrasound transducer, folded transducer). As an example, an ASIC 3504 may be disposed adjacent the transducer 3502. As a further example, inactive piezo elements may be disposed adjacent the transducer 3502. The tip 3500 may include various sensors, control boards, thermistors, and the like. As shown, the tip 3500 includes thermistors 3506 disposed proximal to the transducer 3502. As more clearly shown in FIG. 23, at least a portion of the tip 3500 may be at least partially enclosed by a first material 3700 (e.g., polymer) and at least a portion of the tip 3500 may be at least partially enclosed by a second material 3704. The first and second materials 3700, 3704 may be configured with the same or different characteristics such as differing stiffness (e.g., durometer). As an illustrative example, the first material 3700 may have a higher durometer than the second material 3704. As further illustrated in FIG. 23, a navigation sensor 3702 may be disposed at or adjacent a proximal end of the tip 3500. The navigation sensor 3702 may be in communication with a navigation system to provide tracking and position of the tip 3500 and to provide feedback to a user.

As an illustrative example, FIG. 22 shows the medical tool may comprise a steerable tip 3600 disposed at the distal end of a sheath. The steerable tip 3600 may include a transducer 3602 (e.g., ultrasound transducer, folded transducer). When compared to tip 3500, the tip 3600 may include a shortened folded end 3601. Inactive piezo material may be minimized adjacent the transducer 3602. As an example, an ASIC 3604 may be disposed adjacent the transducer 3602. The tip 3600 may include various sensors, control boards, thermistors, and the like. As shown, the tip 3600 includes thermistors 3606 disposed proximal to the transducer 3602. Circuits such as flex circuits may be in electrical communication with one or more of the components in the tip 3600. As an example, the flex circuit may be divided into layers and selectively in communication with the components. As more clearly shown in FIG. 24, at least a portion of the tip 3600 may be at least partially enclosed by a first material 3800 (e.g., polymer) and at least a portion of the tip 3600 may be at least partially enclosed by a second material 3804. The first and second materials 3800, 3804 may be configured with the same or different characteristics such as differing stiffness (e.g., durometer). As an illustrative example, the first material 3800 may have a higher durometer than the second material 3804. As further illustrated in FIG. 24, a navigation sensor 3802 may be disposed adjacent the transducer 3602. The navigation sensor 3802 may be in communication with a navigation system to provide tracking and position of the tip 3600 and to provide feedback to a user.

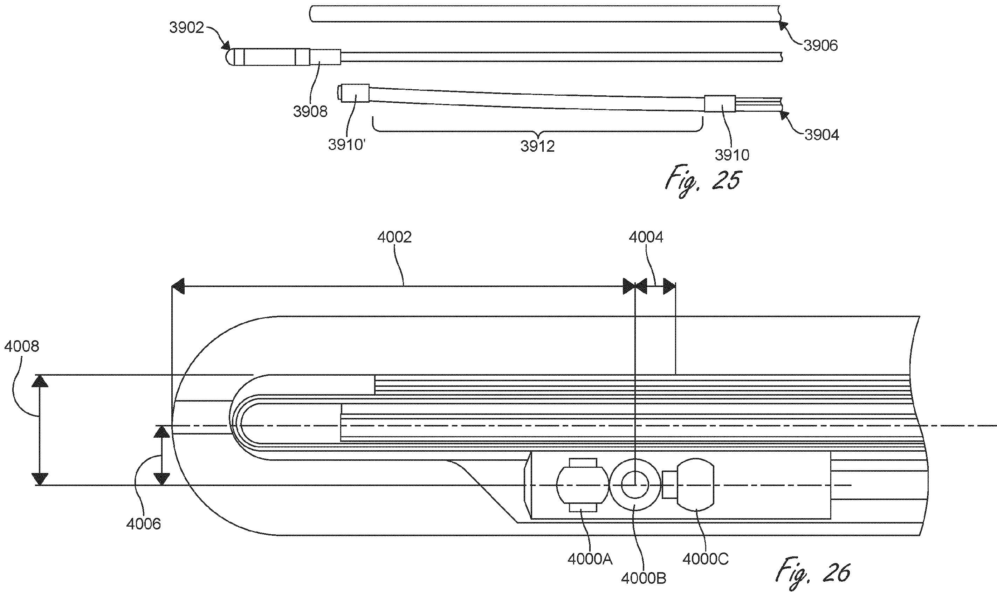

FIG. 25 illustrates a medical tool that may comprise a steerable tip 3902 disposed at the distal end of a sheath 3906. As shown, a DAS sensor 3908 may be disposed at or adjacent a proximal end of the tip 3902. An inner sheath 3904 may comprise a section configured for deflection 3912 and may be disposed adjacent the tip 3902 and within the sheath 3906. One or more DAS sensors 3910, 3910' may be disposed along a length of the sheath 3904.

FIG. 26 illustrates example configurations of navigation transducers 4002. For example, from a tip of the device to a center of a y-coil may be measured at a first length 4003. From the center of an active transducer 4002 to the y-coil may be a second length 4004. From a sensor axis to a tip axis may be a third length 4006. From a sensor axis to a face of an ultrasound transducer may be a fourth length 4008.

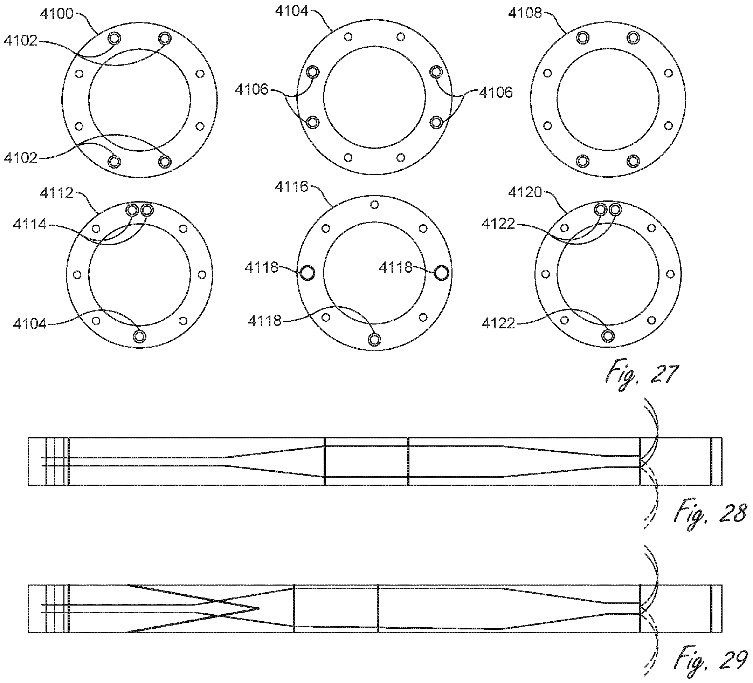

FIGS. 27-29 illustrate various arrangements of pull wires. For example, a handle assembly may have internal components that interface with proximal ends of such pull wires such that actuation of one or more actuators tensions one or more pull wires to cause deflection of an inner shaft. From left to right, the configurations of the inner shaft and wires (showing two versions in vertical pairings for comparison) may represent a distal portion, a mid portion, and a proximal portion along a length of the inner shaft. Wires 4102 may have relative configuration within an outer sheath 4100. Wires 4106 may have relative configuration within an outer sheath 4104. Wires 4110 may have relative configuration within an outer sheath 4108. Wires 4114 may have relative configuration within an outer sheath 4112. Wires 4118 may have relative configuration within an outer sheath 4116. Wires 4122 may have relative configuration within an outer sheath 4120. FIGS. 28-29 illustrate pull wires may change spacing and configuration along the length on one or more shafts or sheaths.

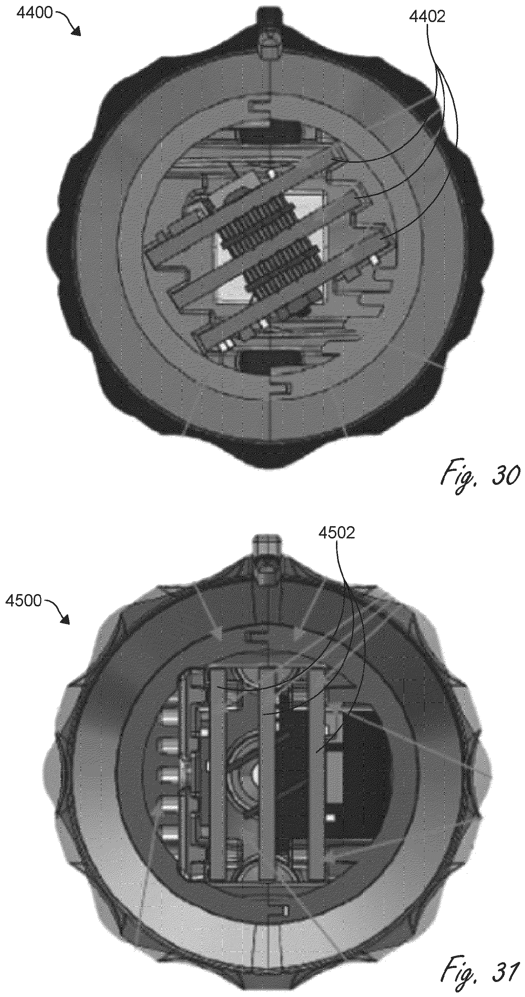

FIGS. 30-31 illustrate various configurations of components of example handle assemblies. As an illustration, handle assembly 4400 may comprise one or more printed circuit boards 4402 in a stacked arrangement. As a further illustration, handle assembly 4500 may comprise one or more printed circuit boards 4502 in a stacked arrangement. Other arrangements may be used.

Medical devices such as catheters are described herein with maneuverability. However, certain procedures may still present difficulties, for example, introducing a medical device to certain regions of the heart, such as through a puncture in the septum and into the left atrium (LA). Such a procedure may require locating the puncture hole and then pushing the medical device (e.g., catheter) through the puncture site. Other procedures and devices may benefit from additional guiding mechanisms and navigation methods.

In an aspect, an over-the-guidewire design is provided, where the distal end of a catheter includes a monorail (e.g., a guidewire lumen). As an illustration, a catheter may have steering capability such as 2-way, or 4-way steering, for example, as described herein, but may benefit from a guidewire. Alternatively, a catheter may have no deflection steering at all, and instead rely solely on advancement over the guidewire. As a further example, a catheter could be advanced within a fixed or steerable sheath.

In certain aspects, a guidewire lumen may comprise a tip extension disposed at or adjacent a distal end of a medical device. The tip extension may be at least partially formed from a soft polymer material (e.g., equivalent to Pebax 25 or 35 durometer). Other materials may be used. The tip extension may comprise a guidewire lumen sized to receive a guidewire therethrough (e.g., a .018" to .035" guidewire; e.g., internal diameter of about .021" to .042"). Other dimensions may be used.

As an illustrative example, to use the device, the guidewire may be placed into the target location on its own, or preferably with the aid of a long introducer sheath or guiding catheter into the heart. Example target locations may include coronary sinus, the pulmonary artery, or across a transseptal hole from the right to left atrium. The catheter would then be advanced over the guidewire to the target location. Alternatively, the catheter and guidewire could be advanced together into the heart and the guidewire advanced forward to the target location before the catheter is tracked over it, for example.

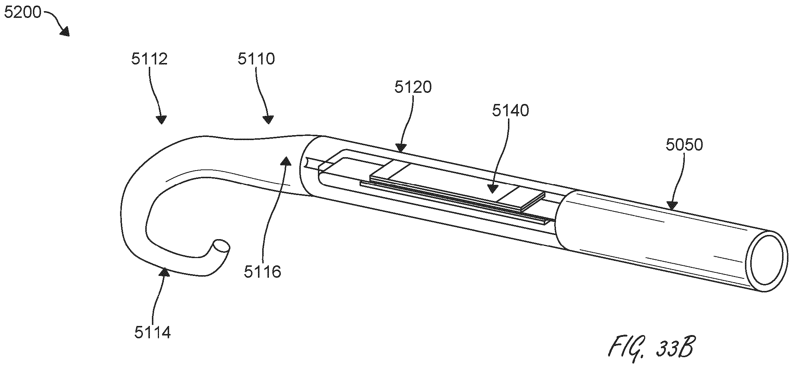

During use, it may be desirable to remove the wire and use the catheter without the aid of the wire. In this case, the tip curling into a "pigtail" type shape (FIG. 33B) would lessen the potential trauma to tissue as the device is manipulated. Alternatively, the catheter may be removed, the wire repositioned, and the catheter again advanced over the back end of the wire to the target location. Other configurations and methods of use may be implemented.

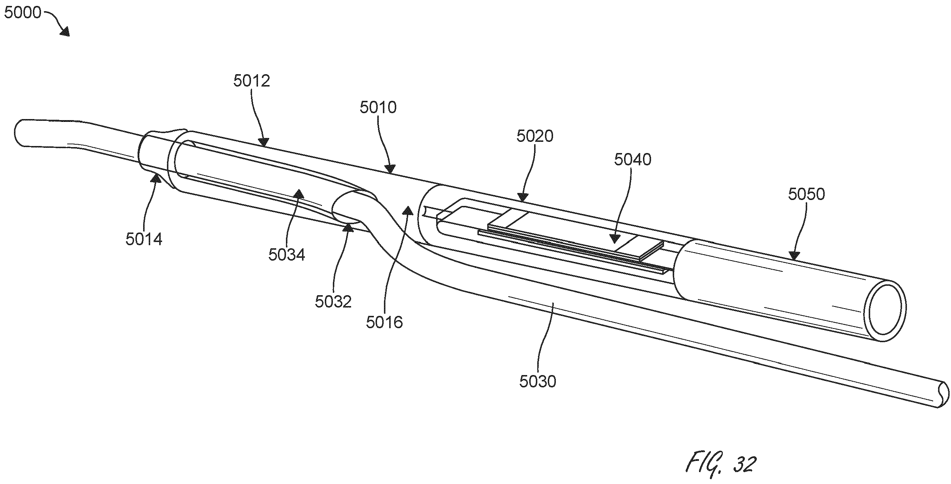

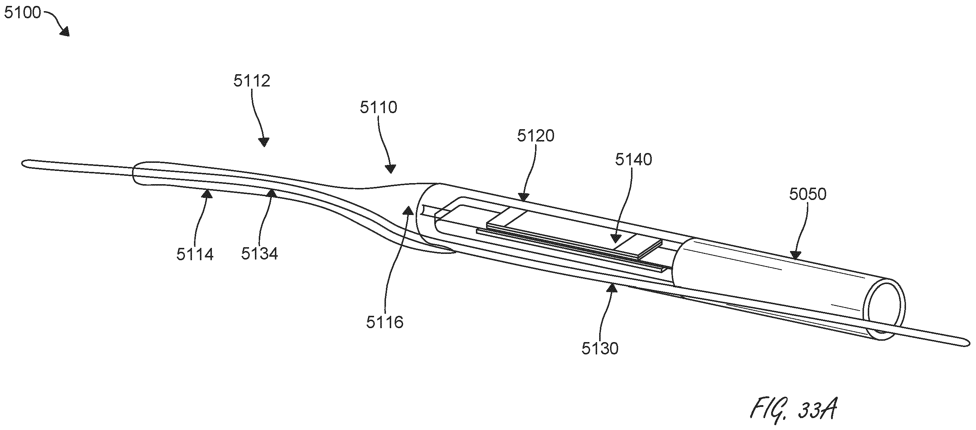

FIGS. 32 and 33A-33B illustrate example medical devices with tip extensions. FIG. 32 shows medical device 5000 comprising a probe 5020, a tip extension 5010, a guidewire 5030, a transducer 5040, and an outer shaft 5050. The tip extension 5010 may comprise a tapered tip 5014, a first (e.g., soft) portion 5012, and a second (e.g., rigid or stiff) portion 5016. Other configurations may be used. The tip extension 5010 may comprise an entrance aperture 5032 to receive the guidewire 5030. The tip extension 5010 may comprise a guidewire lumen 5034. FIG. 33A shows medical device 5100 comprising a probe 5120, a tip extension 5110, a guidewire 5130, a transducer 5140, and an outer shaft 5050. The tip extension 5110 may comprise a tapered tip 5114, a first portion 5112, and a second portion 5116. The first portion may have a stiffness that is less than the second portion. The tip extension 5110 may comprise a guidewire lumen 5134. The guidewire lumen 5134 may align 180 degrees from a face of the transducer 5140 and extend the full length of the medical device 5100. FIG. 33B shows medical device 5200. Medical device 5200 may comprise medical device 5100 (or medical device 5000) after a guidewire (e.g., guidewire 5130 or the guidewire 5030) is removed. As shown, the tip extension 5110 may be configured to take an atraumatic "pigtail" shape when not loaded with the guidewires 5030, 5130. As an illustrative example, the medical devices 5000, 5100, 5200 may comprise a catheter, such as an ICE catheter.

The first (e.g., soft) portions 5012, 5112 may comprise a soft polymer material (e.g., equivalent to Pebax 25 or 35 durometer). The guidewire lumens 5034, 5134 may be sized to a .018 to .035 guidewire (typically ID = .021" to .042"). Stiffness of the tip extensions 5010, 5110 may be more rigid at the distal end of the transducers 5040, 5140 (at second (e.g., stiff) portions 5016, 5116), and soften toward the distal end of the tip extensions 5010, 5110 (at first (e.g., soft) portions 5012, 5112) to better transition to be the same of more closely similar to the guidewires 5030, 5130 stiffness. Stiffness transition may be facilitated by one or more of transitions of outside diameter (or other dimension), internal longitudinal stiffening members, coils, braids, and/or different durometers of materials (e.g., polymer). Other methods and controls for stiffness may be used. Stiffness may transition linearly, or by region/section, or some other transition curve to exhibit a change in stiffness from the distal end of the transducer to the distal end of the tip extension. The tip extensions 5010, 5110 may also be configured to take an atraumatic "pigtail" shape when not loaded with the guidewires 5030, 5130, as shown by medical device 5200.

The guidewire lumens 5034, 5134 may comprise a lubricious liner such as polytetrafluoroethylene (PTFE) or fluorinated ethylene propylene (FEP), or a polyethylene such as high-density polyethylene or blends thereof with low density polyethylene. Other materials may be used. The length of the tip extensions 5010, 5110 may be 1 to 6 cm. In certain aspects, the length of the tip extensions 5010, 5110 may be about 2.5 cm. The length of the guidewire lumens 5034, 5134 may be 0.5-5.5 cm. The length of the guidewire lumens 5034, 5134 may be 2 cm. Other dimensions may be used. The guidewire lumens 5034, 5134 may be distal monorail lumens. In another example, a separate irrigation lumen may be routed through the length of the medical device 5000 (or the medical device 5100), and behind the transducer 5040 (or the transducer 5140), to join the guidewire lumen 5034 (or the guidewire lumen 5134) to keep the guidewire lumen 5034 (or the guidewire lumen 5134) flushed with saline to prevent blood coagulation.

A navigation sensor could be incorporated into a portion (e.g., an end or tip) of the tip extensions 5010, 5110 for accurate display in mapping or navigation technology such as the cardiac 3D mapping system (CARTO) by Biosense Webster. The tip extensions 5010, 5110 may comprise a polymer compounded with a radiopaque material such as barium sulfate or tungsten powder. Radiopaque marker bands, braids, and/or coils comprising platinum and other materials known in the art may also be incorporated into the tip extensions 5010, 5110.

As an example, the guidewire lumen 5134 may extend the full length of the medical device 5100. The proximal end may comprise an attachment for a rotating hemostatic valve to allow infusion of saline through the full length of the guidewire lumen 5134.

To use the medical device 5000 (or the medical device 5100), the guidewire 5030 (or the guidewire 5130) may be placed into a target location on its own. The guidewire 5030 (or the guidewire 5130) may be placed into a target location with the aid of a long introducer sheath or guiding catheter into a heart. Target locations may comprise a Coronary Sinus, a Pulmonary Artery, or across a transseptal hole from a right to left atrium. The medical device 5000 (or the medical device 5100) may then be advanced over the guidewire 5030 (or the guidewire 5130) to a target location. Alternatively, the medical device 5000 (or the medical device 5100) and guidewire 5030 (or guidewire 5130) may be advanced together into a heart and the guidewire 5030 (or the guidewire 5130) advanced forward to a target location before the medical device 5000 (or the medical device 5100) is tracked over the target location.

During use, it may be preferable to remove the guidewire 5030 (or the guidewire 5130) and use the medical device 5000 (or the medical device 5100) without the aid of the guidewire 5030 (or the guidewire 5130). In this case, the tip extension 5010 (or the tip extension 5110) curling into a pigtail type shape, as shown in FIG. 33B, would lessen the potential trauma to tissue as the medical device 5000 (or the medical device 5100) is manipulated. Alternatively, the medical device 5000 (or the medical device 5100) could be removed, the guidewire 5030 (or the guidewire 5130) repositioned, and the medical device 5000 (or the medical device 5100) again advanced over the back end of the guidewire 5030 (or the guidewire 5130) to a target location.

The medical devices 5000, 5100 may comprise steering capability, such as the designs described herein (e.g., 2-way steering, or 4-way steering). The medical devices 5000, 5100 may be manufactured to have no deflection steering, and instead rely on advancement over the guidewires 5030, 5130. The medical devices 5000, 5100 may be advanced within a fixed or steerable sheath.

### EXAMPLE ASPECTS

Example aspect 1: A medical device configured and sized to be positioned within a subject, comprising: an ultrasound transducer disposed in a distal region of the medical device, the ultrasound transducer at least partially enclosed by a material to define a distal tip of the medical device; and a tip extension disposed adjacent the distal tip and extending longitudinally from the distal tip, the tip extension comprising a first end adjacent the distal tip and a second end opposite the first end, wherein the tip extension comprises a tapered tip at or adjacent the second end, wherein the tip extension comprises a guidewire lumen formed therein and configured to receive a guidewire therethrough, and wherein the tip extension exhibits a first stiffness at the first end and a second stiffness adjacent the second end, wherein the first stiffness is greater than the second stiffness.

Example aspect 2: The medical device of aspect 1, further comprising an ultrasound probe comprising the ultrasound transducer and a flexible circuit strip in electrical communication with the ultrasound transducer, the flexible circuit strip further comprising an insulating substrate, a plurality of conductive traces disposed on and extending along the insulating substrate, a portion of one or more of the plurality of conductive traces covered by an insulation member.

Example aspect 3: The medical device of aspect 1, wherein the guidewire lumen comprises one or more lumen aperture configured to allow entrance and removal of a guidewire from the guidewire lumen.

Example aspect 4: The medical device of aspect 1, wherein the guidewire lumen comprises a first lumen aperture disposed along a length of the tip extension and a second lumen aperture disposed at the tapered tip, wherein the first lumen and the second lumen are configured to allow entrance and removal of a guidewire from the guidewire lumen.

Example aspect 5: The medical device of aspect 1, wherein the guidewire lumen extends along at least a part of the length of the ultrasound transducer.

Example aspect 6: The medical device of any one of aspects 1-4, wherein the ultrasound transducer comprises an emitter face and the guidewire lumen extends along at least a part of the length of the ultrasound transducer opposite the emitter face.

Example aspect 7: The medical device of aspect 1, wherein the tip extension is coupled to the distal tip.

Example aspect 8: The medical device of aspect 1, wherein the tip extension is fused to the distal tip.

Example aspect 9: The medical device of any one of aspects 1-8, further comprising an outer shaft coupled to a proximal end of the distal tip.

Example aspect 10: The medical device of aspect 9, wherein the guidewire lumen extends along at least a part of the length of the outer shaft.

Example aspect 11: A medical device configured and sized to be positioned within a subject, comprising: an ultrasound transducer disposed in a distal region of the medical device, the ultrasound transducer at least partially enclosed by a material to define a distal tip of the medical device; and a tip extension disposed adjacent the distal tip and extending longitudinally from the distal tip, the tip extension comprising a first end adjacent the distal tip and a second end opposite the first end, wherein the tip extension comprises a guidewire lumen formed therein and configured to receive a guidewire therethrough, and wherein the tip extension exhibits a transition in stiffness along a length thereof.

Example aspect 12: The medical device of aspect 11, wherein the tip extension exhibits a first stiffness at the first end and a second stiffness adjacent the second end, wherein the first stiffness is greater than the second stiffness.

Example aspect 13: The medical device of any one of aspects 11-12, wherein the tip extension comprises a tapered tip at or adjacent the second end.

Example aspect 14: The medical device of any one of aspects 11-13, further comprising an ultrasound probe comprising the ultrasound transducer and a flexible circuit strip in electrical communication with the ultrasound transducer, the flexible circuit strip further comprising an insulating substrate, a plurality of conductive traces disposed on and extending along the insulating substrate, a portion of one or more of the plurality of conductive traces covered by an insulation member.

Example aspect 15: The medical device of any one of aspects 11-14, wherein the guidewire lumen comprises one or more lumen aperture configured to allow entrance and removal of a guidewire from the guidewire lumen.

Example aspect 16: The medical device of any one of aspects 11-14, wherein the guidewire lumen comprises a first lumen aperture disposed along a length of the tip extension and a second lumen aperture disposed adjacent an end of the tip extension, wherein the first lumen and the second lumen are configured to allow entrance and removal of a guidewire from the guidewire lumen.

Example aspect 17: The medical device of any one of aspects 11-16, wherein the guidewire lumen extends along at least a part of the length of the ultrasound transducer.

Example aspect 18: The medical device of any one of aspects 11-16, wherein the ultrasound transducer comprises an emitter face and the guidewire lumen extends along at least a part of the length of the ultrasound transducer opposite the emitter face.

Example aspect 19: The medical device of any one of aspects 11-16, wherein the tip extension is coupled to the distal tip.

Example aspect 20: The medical device of any one of aspects 11-16, wherein the tip extension is fused to the distal tip.

Example aspect 21: The medical device of any one of aspects 11-20, further comprising an outer shaft coupled to a proximal end of the distal tip.

Example aspect 22: The medical device of aspect 21, wherein the guidewire lumen extends along at least a part of the length of the outer shaft.

Example aspect 23: A medical device configured and sized to be positioned within a subject, comprising: an ultrasound probe disposed adjacent a distal region of a rotatable shaft, the ultrasound probe comprising an ultrasound transducer; a deflectable shaft disposed relative to the rotatable shaft such that deflection of the deflectable shaft causes deflection of at least a portion of the rotatable shaft; a handle assembly comprising a handle body with an outer surface that can be gripped by a user, a first actuator configured to be moved relative to the handle body, and a second actuator configured to be moved relative to the handle body, wherein the first actuator and the second actuator are circumferentially disposed about a longitudinal axis of the handle body, wherein the rotatable shaft is in operable communication with the first actuator of the handle assembly such that actuation of the first actuator causes rotational movement of at least a portion of the rotatable shaft relative to the deflectable shaft, and wherein the deflectable shaft is in operable communication with the second actuator such that rotation of the second actuator causes deflection of the deflectable shaft and thereby deflection of the rotatable shaft; and a tip extension disposed adjacent the ultrasound probe and extending longitudinally from the ultrasound probe, the tip extension comprising a first end adjacent the ultrasound probe and a second end opposite the first end, wherein the tip extension comprises a guidewire lumen formed therein and configured to receive a guidewire therethrough, and wherein the tip extension exhibits a transition in stiffness along a length thereof.

Example aspect 24: The medical device of aspect 23, wherein the tip extension exhibits a first stiffness at the first end and a second stiffness adjacent the second end, wherein the first stiffness is greater than the second stiffness.

Example aspect 25: The medical device of any one of aspects 23-24, wherein the tip extension comprises a tapered tip at or adjacent the second end.

Example aspect 26: The medical device of any one of aspects 23-25, wherein the ultrasound probe comprises a flexible circuit strip in electrical communication with the ultrasound transducer, the flexible circuit strip further comprising an insulating substrate, a plurality of conductive traces disposed on and extending along the insulating substrate, a portion of one or more of the plurality of conductive traces covered by an insulation member.

Example aspect 27: The medical device of any one of aspects 23-26, wherein the guidewire lumen comprises one or more lumen aperture configured to allow entrance and removal of a guidewire from the guidewire lumen.

Example aspect 28: The medical device of any one of aspects 23-26, wherein the guidewire lumen comprises a first lumen aperture disposed along a length of the tip extension and a second lumen aperture disposed adjacent an end of the tip extension, wherein the first lumen and the second lumen are configured to allow entrance and removal of a guidewire from the guidewire lumen.

Example aspect 29: The medical device of any one of aspects 23-28, wherein the guidewire lumen extends along at least a part of the length of the ultrasound transducer.

Example aspect 30: The medical device of any one of aspects 23-28, wherein the ultrasound transducer comprises an emitter face and the guidewire lumen extends along at least a part of the length of the ultrasound transducer opposite the emitter face.

Example aspect 31: The medical device of any one of aspects 23-30, wherein the tip extension is coupled to the ultrasound probe.

Example aspect 32: The medical device of any one of aspects 23-30, wherein the tip extension is fused to the ultrasound probe.

Example aspect 33: The medical device of any one of aspects 23-32, further comprising an outer shaft coupled to a proximal end of the ultrasound probe.

Example aspect 34: The medical device of aspect 33, wherein the guidewire lumen extends along at least a part of the length of the outer shaft.

Example aspect 35: A medical device configured and sized to be positioned within a subject, comprising: a probe tip extension configured to be coupled to a tip of an ultrasound probe configured and sized to be positioned within a subject, wherein the probe tip extension is configured to extended longitudinally from the tip, the tip extension comprising a first end adjacent the tip and a second end opposite the first end, and wherein the probe tip extension exhibits a transition in stiffness along a length thereof; and a guidewire lumen formed through the probe tip extension and configured to receive a guidewire therethrough such that the ultrasound probe may be move along a length of the guidewire via the guidewire lumen.

Example aspect 36: The medical device of aspect 35, wherein the guidewire lumen comprises one or more lumen aperture configured to allow entrance and removal of a guidewire from the guidewire lumen.

Example aspect 37: The medical device of aspect 35, wherein the guidewire lumen comprises a first lumen aperture disposed along a length of the tip extension and a second lumen aperture disposed adjacent and end of the tip extension, wherein the first lumen and the second lumen are configured to allow entrance and removal of a guidewire from the guidewire lumen.

Example aspect 38: The medical device of aspect 35, wherein the tip extension exhibits a first stiffness at the first end and a second stiffness adjacent the second end, wherein the first stiffness is greater than the second stiffness.

Example aspect 39: The medical device of aspect 35, wherein the tip extension comprises a tapered tip at or adjacent the second end.

Example aspect 40: The medical device of any one of aspects 1-39, wherein the guidewire lumen is disposed distally of the ultrasound transducer.

Example aspect 41: The medical device of any one of aspects 1-39, wherein the guidewire lumen is disposed completely distally of the ultrasound transducer.

Regardless of the reference number with which they are labeled, any of the handle assemblies, medical tools, steerable sheaths, and electrical connections herein can be used together in a system in any combination with each other.

Any of the technology, including ultrasound and steering technology, in any of the following U.S. patent references may be incorporated into any of the medical tools, devices, systems, or methods of use thereof herein, the disclosures of which are incorporated by reference herein: 6100626, 6537217, 6559389, 7257051, 7297118, 7331927, 7338450, 7451650, 7451650, 7527591, 7527592, 7569015, 7621028, 7731516, 7740584, 7766833, 7783339, 7791252, 7791252, 7819802, 7824335, 7966058, 8057397, 8096951, 8207652, 8207652, 8213693, 8364242, 8428690, 8451155, 8527032, 8659212, 8721553, 8727993, 8742646, 8742646, 8776335, 8790262, 8933613, 8978216, 8989842, 9055883, 9439625, 9575165, 9639056, and 20080287783.

### Aspects of invention:

1. A medical device configured and sized to be positioned within a subject, comprising:
   an ultrasound transducer disposed in a distal region of the medical device, the ultrasound transducer at least partially enclosed by a material to define a distal tip of the medical device; and
   a tip extension disposed adjacent the distal tip and extending longitudinally from the distal tip, the tip extension comprising a first end adjacent the distal tip and a second end opposite the first end,
      wherein the tip extension comprises a tapered tip at or adjacent the second end,
      wherein the tip extension comprises a guidewire lumen formed therein and configured to receive a guidewire therethrough, and
      wherein the tip extension exhibits a first stiffness at the first end and a second stiffness adjacent the second end, wherein the first stiffness is greater than the second stiffness.
2. The medical device of aspect 1, further comprising an ultrasound probe comprising the ultrasound transducer and a flexible circuit strip in electrical communication with the ultrasound transducer, the flexible circuit strip further comprising an insulating substrate, a plurality of conductive traces disposed on and extending along the insulating substrate, a portion of one or more of the plurality of conductive traces covered by an insulation member.
3. The medical device of aspect 1, wherein the guidewire lumen comprises one or more lumen aperture configured to allow entrance and removal of a guidewire from the guidewire lumen.
4. The medical device of aspect 1, wherein the guidewire lumen comprises a first lumen aperture disposed along a length of the tip extension and a second lumen aperture disposed at the tapered tip, wherein the first lumen aperture and the second lumen aperture are configured to allow entrance and removal of a guidewire from the guidewire lumen.
5. The medical device of aspect 1, wherein the guidewire lumen extends along at least a part of a length of the ultrasound transducer.
6. The medical device of aspect 1, wherein the ultrasound transducer comprises an emitter face and the guidewire lumen extends along at least a part of a length of the ultrasound transducer opposite the emitter face.
7. The medical device of aspect 1, wherein the tip extension is coupled to the distal tip.
8. The medical device of aspect 1, wherein the tip extension is fused to the distal tip.
9. The medical device of aspect 1, further comprising an outer shaft coupled to a proximal end of the distal tip.
10. The medical device of aspect 9, wherein the guidewire lumen extends along at least a part of a length of the outer shaft.
11. A medical device configured and sized to be positioned within a subject, comprising:
   an ultrasound probe disposed adjacent a distal region of a rotatable shaft, the ultrasound probe comprising an ultrasound transducer;
   a deflectable shaft disposed relative to the rotatable shaft such that deflection of the deflectable shaft causes deflection of at least a portion of the rotatable shaft;
   a handle assembly comprising a handle body with an outer surface that can be gripped by a user, a first actuator configured to be moved relative to the handle body, and a second actuator configured to be moved relative to the handle body,
   wherein the first actuator and the second actuator are circumferentially disposed about a longitudinal axis of the handle body,
   wherein the rotatable shaft is in operable communication with the first actuator of the handle assembly such that actuation of the first actuator causes rotational movement of at least a portion of the rotatable shaft relative to the deflectable shaft, and
   wherein the deflectable shaft is in operable communication with the second actuator such that rotation of the second actuator causes deflection of the deflectable shaft and thereby deflection of the rotatable shaft; and
   a tip extension disposed adjacent a distal end of the ultrasound probe and extending longitudinally from the ultrasound probe, the tip extension comprising a first end adjacent the ultrasound probe and a second end opposite the first end,
   wherein the tip extension comprises a guidewire lumen formed therein and configured to receive a guidewire therethrough, and
   wherein the tip extension exhibits a transition in stiffness along a length thereof.
12. The medical device of aspect 11, wherein the tip extension exhibits a first stiffness at the first end and a second stiffness adjacent the second end, wherein the first stiffness is greater than the second stiffness.
13. The medical device of aspect 11, wherein the tip extension comprises a tapered tip at or adjacent the second end.
14. The medical device of aspect 11, wherein the ultrasound probe comprises a flexible circuit strip in electrical communication with the ultrasound transducer, the flexible circuit strip further comprising an insulating substrate, a plurality of conductive traces disposed on and extending along the insulating substrate, a portion of one or more of the plurality of conductive traces covered by an insulation member.
15. The medical device of aspect 11, wherein the guidewire lumen comprises one or more lumen aperture configured to allow entrance and removal of a guidewire from the guidewire lumen.
16. The medical device of aspect 11, wherein the guidewire lumen comprises a first lumen aperture disposed along a length of the tip extension and a second lumen aperture disposed adjacent an end of the tip extension, wherein the first lumen aperture and the second lumen aperture are configured to allow entrance and removal of a guidewire from the guidewire lumen.
17. The medical device of aspect 11, wherein the guidewire lumen extends along at least a part of the length of the ultrasound transducer.
18. The medical device of aspect 11, wherein the ultrasound transducer comprises an emitter face and the guidewire lumen extends along at least a part of the length of the ultrasound transducer opposite the emitter face.
19. The medical device of aspect 11, wherein the tip extension is coupled to the ultrasound probe.
20. The medical device of aspect 11, wherein the tip extension is fused to the ultrasound probe.
21. The medical device of aspect 11, further comprising an outer shaft coupled to a proximal end of the ultrasound probe.
22. The medical device of aspect 21, wherein the guidewire lumen extends along at least a part of the length of the outer shaft.
23. A medical device configured and sized to be positioned within a subject, comprising:
   a probe tip extension configured to be coupled to a tip of an ultrasound probe configured and sized to be positioned within a subject, wherein the probe tip extension is configured to extended longitudinally from the tip, the probe tip extension comprising a first end adjacent the tip and a second end opposite the first end, and wherein the probe tip extension exhibits a transition in stiffness along a length thereof; and
   a guidewire lumen formed through the probe tip extension and configured to receive a guidewire therethrough such that the ultrasound probe may be move along a length of the guidewire via the guidewire lumen.
24. The medical device of aspect 23, wherein the guidewire lumen comprises one or more lumen aperture configured to allow entrance and removal of a guidewire from the guidewire lumen.
25. The medical device of aspect 23, wherein the guidewire lumen comprises a first lumen aperture disposed along a length of the probe tip extension and a second lumen aperture disposed adjacent and end of the probe tip extension, wherein the first lumen aperture and the second lumen aperture are configured to allow entrance and removal of a guidewire from the guidewire lumen.
26. The medical device of aspect 23, wherein the probe tip extension exhibits a first stiffness at the first end and a second stiffness adjacent the second end, wherein the first stiffness is greater than the second stiffness.
27. The medical device of aspect 23, wherein the probe tip extension comprises a tapered tip at or adjacent the second end.

## Claims

1. A medical device configured and sized to be positioned within a subject, comprising:
an ultrasound transducer disposed in a distal region of the medical device, the ultrasound transducer at least partially enclosed by a material to define a distal tip of the medical device; and
a tip extension disposed adjacent the distal tip and extending longitudinally from the distal tip, the tip extension comprising a first end adjacent the distal tip and a second end opposite the first end,
wherein the tip extension comprises a guidewire lumen formed therein and configured to receive a guidewire therethrough, and
wherein the tip extension exhibits a transition in stiffness along a length thereof.

2. The medical device of claim 1, wherein the tip extension exhibits a first stiffness at the first end and a second stiffness adjacent the second end, wherein the first stiffness is greater than the second stiffness.

3. The medical device of claim 1, wherein the tip extension comprises a tapered tip at or adjacent the second end.

4. The medical device of claim 1, further comprising an ultrasound probe comprising the ultrasound transducer and a flexible circuit strip in electrical communication with the ultrasound transducer, the flexible circuit strip further comprising an insulating substrate, a plurality of conductive traces disposed on and extending along the insulating substrate, a portion of one or more of the plurality of conductive traces covered by an insulation member.

5. The medical device of claim 1, wherein the guidewire lumen comprises one or more lumen aperture configured to allow entrance and removal of a guidewire from the guidewire lumen.

6. The medical device of claim 1, wherein the guidewire lumen comprises a first lumen aperture disposed along a length of the tip extension and a second lumen aperture disposed adjacent an end of the tip extension, wherein the first lumen aperture and the second lumen aperture are configured to allow entrance and removal of a guidewire from the guidewire lumen.

7. The medical device of claim 1, wherein the guidewire lumen extends along at least a part of the length of the ultrasound transducer.

8. The medical device of claim 1, wherein the ultrasound transducer comprises an emitter face and the guidewire lumen extends along at least a part of the length of the ultrasound transducer opposite the emitter face.

9. The medical device of claim 1, wherein the tip extension is coupled to the distal tip.

10. The medical device of claim 1, wherein the tip extension is fused to the distal tip.

11. The medical device of claim 1, further comprising an outer shaft coupled to a proximal end of the distal tip.

12. The medical device of claim 11, wherein the guidewire lumen extends along at least a part of the length of the outer shaft.
